(19) Europäisches Patentamt
European Patent Office
Office européen des brevets

(11) Veröffentlichungsnummer: **0 324 474 B1**

(12) # EUROPÄISCHE PATENTSCHRIFT

(45) Veröffentlichungstag der Patentschrift: **31.03.93**

(51) Int. Cl.$^5$: **C12Q 1/68**, C07H 21/00

(21) Anmeldenummer: **89100482.2**

(22) Anmeldetag: **12.01.89**

Die Akte enthält technische Angaben, die nach dem Eingang der Anmeldung eingereicht wurden und die nicht in dieser Patentschrift enthalten sind.

(54) **Verfahren zum Nachweis von Nukleinsäuren.**

(30) Priorität: **12.01.88 DE 3800642**
**20.04.88 DE 3813278**

(43) Veröffentlichungstag der Anmeldung:
**19.07.89 Patentblatt 89/29**

(45) Bekanntmachung des Hinweises auf die Patenterteilung:
**31.03.93 Patentblatt 93/13**

(84) Benannte Vertragsstaaten:
**AT BE CH DE ES FR GB GR IT LI LU NL SE**

(56) Entgegenhaltungen:
**EP-A- 63 879**
**EP-A- 173 251**

**CHEMICAL ABSTRACTS, Band 110, Nr. 5, 30 Jänner 1989; R.SCHAEFER et al., Seite 159, Nr. 34785g***

**BIOLOGICAL ABSTRACTS, Band 87, 1989; H.B.J.HEILES et al., Nr. 87049454***

**BIOLOGICAL ABSTRACTS RRM, Band 36, 1989; S.DOOLEY et al., Nr. 36065581***

(73) Patentinhaber: **BOEHRINGER MANNHEIM GMBH**
**Sandhofer Strasse 112-132**
**W-6800 Mannheim-Waldhof(DE)**

(72) Erfinder: **Höltke, Hans Joachim, Dr.**
**Kirchenstr. 1**
**W-8132 Tutzing(DE)**
Erfinder: **Seibl, Rudolf, Dr.**
**Saalangerstr. 46**
**W-8122 Penzberg(DE)**
Erfinder: **Schmitz, Gudrun, Dr.**
**Wettersteinstr. 3**
**W-8139 Bernried(DE)**
Erfinder: **Schöler, Hans Robert, Dr.**
**Lotzestr. 40**
**W-3400 Göttingen(DE)**
Erfinder: **Kessler, Christop, Dr.**
**Fraunhofer Str. 12**
**W-8000 München 5(DE)**
Erfinder: **Mattes, Ralf, Prof. Dr.**
**Friedrich Zundel-Str. 14**
**W-7000 Stuttgart 75(DE)**

**EP 0 324 474 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

VIROLOGY, Band 126, 1983, New York, NY (US); D.J.BRIGATI et al., Seiten 32-50*

NUCLEIC ACIDS RESEARCH, Band 13, Nr. 3, 1985, London (GB); A.C.FORSTER et al., Seiten 745-761*

P. N. A. S., Band 70, Nr. 8, 1973; Seiten 2238-2242*

## Beschreibung

Die Erfindung betrifft ein Verfahren zum Nachweis von Nukleinsäuren definierter Sequenz durch Hybridisierung mit einer komplementären, markierten Nukleinsäure-Sonde.

Eine der meist angewandten molekular-biologischen Techniken ist die DNA/DNA-, RNA/RNA- bzw. RNA/DNA-Hybridisierung zum Nachweis homologer Nukleinsäure-Sequenzen. Dabei wird eine als Sonde verwendete Nukleinsäure (DNA oder RNA) markiert und mit einer meist auf einem Filter fixierten, zu untersuchenden Nukleinsäure (DNA oder RNA) unter Hybridisierungsbedingungen in Kontakt gebracht. Bei vorhandener Homologie zwischen der als Sonde verwendeten Nukleinsäuren und der nachzuweisenden Nukleinsäure kommt es sodann zur Hybridisierung der jeweils komplementären Nukleinsäure-Einzelstränge unter Ausbildung eines Hybrid-Doppelstranges. Anschließend werden die Hybride nachgewiesen. Bisher erfolgte die Markierung der Sonde meist durch Einbau radioaktiv derivatisierter Desoxyribonukleosidtriphosphate. Der Nachweis der Hybride erfolgte dann durch Autoradiographie. Solche konventionellen, radioaktiv markierten DNA-Sonden sind sehr effektiv und sensitiv, es ergeben sich jedoch durch den Umgang mit der Radioaktivität Probleme. So wird zum Umgang mit den radioaktiven Verbindungen speziell ausgebildetes Personal benötigt, da bei unsachgemäßer Handhabung eine Gefährdung der Laborsicherheit besteht. Außerdem bildet die Entsorgung der radioaktiven Verbindungen ein weiteres Problem. Zusätzlich sind die radioaktiv markierten Proben aufgrund der Halbwertzeit der verwendeten radioaktiven Materialien nur über einen gewissen Zeitraum nach der Herstellung verwendbar. Auch kann bei der Detektion von geringen Mengen von nachzuweisender DNA die nötige Belichtungszeit der Autoradiographie sehr lange, nämlich Tage bis Wochen sein.

Neben den radioaktiv markierten Systemen zum Nachweis von Nukleinsäuren sind nicht-radioaktive Methoden bekannt, wobei die verwendeten Nukleinsäure-Proben mit Biotin-Molekülen (US-PS 2 915 082, EP-A 0063879) Digoxin-/T3-/T4-Molekülen (EP-A-O 173251) oder mit Alkyl-/Butyl-/Ethyl-/Sulfonsäure-/Nitrosomolekülen EP-A 128018) modifiziert sind. Der Einbau dieser niedermolekularen Nachweismoleküle in die komplementäre Nukleinsäure-Sonde erfolgt dabei chemisch, photochemisch oder enzymatisch. Sodann erfolgt die Hybridisierung mit der nachzuweisenden Nukleinsäuresequenz. Der Nachweis der Hybride erfolgt dann über Bindung des niedermolekularen Moleküls durch ein (Strept)-Avidin-Markerenzym-Konjugat im Fall von Biotin, Antidigoxin/-T3-/-T4-Antikörper-Markerenzym-Konjugate im Fall von Digoxin-/T3-/T4-Molekülen oder über Anti-Alkyl-/-Butyl-/-Ethyl-/-Sulfonsäure-/-Nitroso-Antikörper-Markerenzym-Konjugate. Der Nachweis des Hybridisierungsproduktes erfolgt durch die Bestimmung der enzymatischen Aktivität des Markerenzyms über gekoppelte Farbstoffsysteme. Bei der Methode der EP-A-0 173 251 erfolgt jedoch die Bindung der Digoxin/T3-/T4-Moleküle an ein an der Wasserstoffbrückenbindung beteiligtes N-Atom einer oder mehrerer Basen der Nukleinsäuresonde, wodurch die Hybridisierung - vor allem bei Mehrfachmodifizierung der Sonde - mit der nachzuweisenden Nukleinsäure beeinträchtigt sein kann. Mit Ausnahme des Biotin/(Strept)-Avidin-System ist die Sensitivität der derzeit bekannten, nicht-radioaktiven Systeme, im Vergleich mit radioaktiven Systemen, jedoch um mindestens den Faktor 10 bis 100 vermindert. Die bisher einmalig hohe Sensitivität des nicht-radioaktiven Nachweises auf Biotin/(Strept)-Avidin-Basis ist auf die hohe Bindungskonstante ($K = 10^{15}$ $Mol^{-1}$) speziell des Biotin/(Strept)-Avidin-Systems zurückzuführen (Lit. Kinow; Proc. Natl. Acad. Sci. USA 80 (1983) 4045). Die maximal erreichbare Sensitivität des Biotin/(Strept)-Avidin-Systems liegt wie auch bei radioaktiver Markierung im Nachweis von 1 pg bis 0,1 pg DNA im Dot-Blot, und im Nachweis von "single-copy" Genen, das heißt von nur einmal im Genom vorkommenden Genen, im genomischen Blot bei Verwendung von 10 bis 1 µg genomischer DNA-Fragmente. Die Ausnützung der Biotin/(Strept-Avidin-Wechselwirkung hat jedoch den entscheidenden Nachteil, daß sie sehr störanfällig ist, da das Vitamin Biotin in nahezu allen biologischen Materialien vorkommt (Biochem. Biophys. Acta 29 (1985) 225; Biochem. Biophys. Acta 41 (1960) 122).

Die Aufgabe der Erfindung bestand daher darin, ein Verfahren zum Nachweis von Nukleinsäuren bereitzustellen, das die Verwendung einer nicht radioaktiven Markierung erlaubt und weniger störanfällig als die Biotin/(Strept)-Avidin-Wechselwirkung ist, andererseits jedoch die bisher einzigartig hohe Nachweis-Sensitivität der radioaktiven Markierung bzw. der Biotin/(Strept)-Avidin-Markierung erreicht.

Diese Aufgabe wird erfindungsgemäß gelöst durch ein Verfahren zum Nachweis von Nukleinsäuren definierter Sequenz durch Hybridisierung mit einer komplementären Nukleinsäuresonde, die über eine chemische Verbindung mindestens ein Hapten als Markierung gebunden enthält, welches dadurch gekennzeichnet ist, daß man als Hapten ein Steroid verwendet, das an mindestens eine Position der Nukleinsäure, die nicht an der Wasserstoffbrückenbildung beteiligt ist, über eine Brücke von mindestens 4 Atomen Länge gebunden ist und die hybridisierte Probe über einen seinerseits markierten Anti-Hapten-Antikörper nachweist.

Ebenfalls Gegenstand der Erfindung sind derivatisierte Nukleinsäuren, die als Sonden in dem genannten Verfahren eingesetzt werden können, deren Verwendung in den genannten Verfahren, Verfahren zum Nachweis von Nukleinsäuren, bei denen entsprechend markierte Mononukleosidtriphosphate verwendet werden, diese Mononukleosidtriphosphate selbst, sowie ein enzymatisches und ein chemisches Verfahren zur Herstellung von oben genannten derivatisierten Nukleinsäuren.

Bevorzugt werden hierbei als Steroid Digoxigenin oder Digoxin verwendet.

Das erfindungsgemäße Verfahren ermöglicht den Nachweis von 0,5 pg bis 0,05 pg homologer DNA im Dot-Blot und von "single copy"-Genen in 5 µg bis 0,5 µg genomischen DNA-Fragmenten. In beiden Nachweisarten ist die Nachweissensitivität mindestens analog der Nachweissensitivität des Biotin/(Strept)-Avidin-Systems. Dies ist insofern überraschend, da die Bindungskonstante der Biotin/(Strept)-Avidin-Wechselwirkung ($K = 10^{15}$ $Mol^{-1}$, Green, N.M. (1975) Adv. Protein Chem. 29, 85-133; Chaiet, L., Wolf, F.J. (1964) Arch. Biochem. Biophys. 106, 1-5) um mindestens den Faktor $10^5$ höher ist als die Wechselwirkung von Digoxigenin oder Digoxin mit dem entsprechenden Antikörper ($K = 2x10^8$ $Mol^{-1}$ - $7x10^9$ $Mol^{-1}$, Hunter et al., J. Immunol. Vol. 129, Nr. 3 (1982) 1165), und zusätzlich die Biotin/(Strept)-Avidin-Wechselwirkung durch das Vorhandensein von 4 Biotin-Bindungsstellen auf (Strept)-Avidin begünstigt ist.

Ein weiterer überraschender technischer Vorteil ist, daß bei Verwendung von Digoxigenin oder Digoxin und den dazugehörigen Antikörpern sehr viel weniger unspezifische Bindung (background) an den Filter, auf welchem die nachzuweisende Nukleinsäure fixiert ist, entsteht, als bei Verwendung von Biotin/(Strept)-Avidin.

Die Durchführung des erfindungsgemäßen Verfahrens zum Nachweis von Nukleinsäuren läßt sich in drei Schritte aufgliedern.

1. Die Derivatisierung der als Nachweisreagenz dienenden Nukleinsäure-Probe mit dem Hapten,
2. die Hybridisierung, und
3. die Detektion der Hybride.

Zur Derivatisierung von Nukleinsäuren (DNA und RNA) können verschiedene Methoden angewendet werden (Molecular Cloning, Maniatis et al., (1982) Cold Spring Harbor Laboratory, Cold Spring Harbor, New York). Der Einbau des Haptens kann hierbei enzymatisch, chemisch oder photochemisch erfolgen

In der "Random-primed" Methode (Anal. Biochem. 132 (1983) 6) werden doppelsträngige Desoxyribonukleinsäure-Sonden zunächst denaturiert, d.h. die beiden Stränge werden durch Erhitzen getrennt und dadurch in Nukleinsäure-Einzelstränge überführt. Bei einzelsträngigen Desoxyribonukleinsäuresonden entfällt die Denaturierung. An die Desoxyribonukleinsäure-Einzelstränge werden sogenannte "Random Primer", das sind beliebige kurze Oligo-Desoxyribonukleotide mit unterschiedlichen Sequenzen, die mit komplementären Sequenzabschnitten der Einzelstrang-DNA hybridisieren, gebunden. Anschließend wird ausgehend von den 3′-OH-Enden der "Random Primern" durch Einwirkung des Enzyms Klenow Polymerase oder anderer DNA-Polymerasen (z.B. Phage T4 oder T7-kodiert oder aus Thermus aquaticus) der zum Einzelstrang komplementäre Strang synthetisiert. Dabei werden die als Substrat angebotenen vier Desoxyribonukleosidtriphosphat-Arten eingebaut. Bis zu vier dieser Triphosphat-Arten sind teilweise oder gänzlich durch Kopplung eines Steroids über eine Brücke derivatisiert, so daß bei der Desoxyribonukleinsäure-Synthese dieses Steroid miteingebaut wird. Ein bevorzugtes, Steroid-derivatisiertes Nukleosidtriphosphat ist in der Fig. 1 mit zwei verschiedenen bevorzugten Brückenkettenlängen dargestellt.

In der "Specific-primed" Methode werden anstelle von "Random Primern", also kurzen Oligo-Desoxyribonukleotiden mit unterschiedlichsten Sequenzen, "Specific Primer", also Oligo-Desoxyribonukleotide mit spezifischen Sequenzen, verwendet. Diese spezifischen Primer binden einheitlich nur an den komplementären Sequenzabschnitt der Einzelstrang-DNA. Die Synthese des komplementären Stranges wird im Gegensatz zur "Random-primer"-Methode nur von diesem bestimmten Sequenzabschnitt gestartet. Es werden die als Substrat angebotenen vier Desoxyribonukleosidtriphosphat-Arten eingebaut. Bis zu vier dieser Triphosphat-Arten sind teilweise oder gänzlich durch Kopplung eines Steroids über eine Brücke derivatisiert, so daß bei der Desoxyribonukleinsäure-Synthese dieses Steroid mit eingebaut wird.

Bei der "Reversen Transkriptions" Methode (Efstratiadis, A.F.C., Villa-Komaroff, L. (1979) Genetic Engeneering (Stelow, J.K. and Hollaender, A., eds.) Plenum Press, New York and London, Vol. 1, pp. 1) werden einzelsträngige Ribonukleinsäure-Sonden oder doppelsträngige Ribonukleinsäure-Sonden nach Denaturierung, d.h. Überführung in Einzelstränge, retrotranskribiert, d.h. die entsprechende Desoxyribonukleinsäure gebildet. Hierzu werden an die einzelsträngigen Ribonukleinsäure-Sonden Oligo- Desoxyribonukleotide, die "Primer", an den komplementären Sequenzabschnitt gebunden. Anschließend wird, ausgehend von dem 3′-OH-Ende des gebundenen "Primers" durch Einwirkung des Enzyms Reverse Transkriptase der zum RNA-Einzelstrang komplementäre DNA-Strang synthetisiert. Als Reverse Transkriptasen werden z.B. Virus AMV oder Mo-MLV kodierte Enzyme verwendet. Bei der DNA-Strangsynthese werden die als Substrat angebotenen vier Desoxyribonukleosidtriphosphat-Arten eingebaut. Bis zu vier dieser Triphosphat-Arten sind teilweise oder gänzlich durch Kopplung eines Steroids über eine Brücke derivatisiert, so daß bei der DNA-Synthese dieses Steroid mit eingebaut wird.

In der "Fill-in" Methode werden doppelsträngige Desoxyribonukleinsäure-Sonden mit 5′-überhängenden Einzelstrang-Enden derivatisiert. Dazu werden mit dem Enzym Klenow Polymerase oder anderer DNA-Polymerasen (z.B. Phage T4-oder T7- codiert) die 3′-OH Enden des nicht-überstehenden Einzelstrangs komplementär zu der 5′-überhängenden Einzelstrang-Sequenz verlängert. Dabei werden, je nach Sequenz des 5′-überhängenden Einzelstrang-Bereichs, bis zu vier der angebotenen Desoxyribonukleosidtriphosphat-Arten eingebaut. Bis zu vier dieser Triphosphat-Arten sind wiederum teilweise oder gänzlich durch Kopplung eines Steroids über eine Brücke derivatisiert, so daß bei dem Einbau der Nukleotide dieses Steroid mit eingebaut wird.

In der "Nick-Translation" Methode (J. Mol. Biol. 113 (1977) 237) werden doppelsträngige Desoxyribonukleinsäure-Sonden gleichzeitig mit dem Enzym E. coli DNA Polymerase I sowie einer kleinen Menge des Enzyms DNase I in Anwesenheit der als Substrat dienenden vier Desoxyribonukleosidtriphosphate inkubiert. Durch das Enzym DNase I werden Einzelstrang-Brüche, die sogenannten "nicks", erzeugt. Dadurch entstehen 5′- und 3′-Enden innerhalb des gebrochenen Einzelstranges. Durch das Enzym E. coli DNA Polymerase I werden anschließend an den internen Einzelstrang-Brüchen die 5′-endständigen Desoxyribonukleoside abgebaut, gleichzeitig jedoch an das benachbarte freie 3′-OH-Ende eines der als Substrat angebotenen Desoxyribonukleotid-Arten eingebaut. Durch wiederholtes Ablaufen des gleichzeitigen Ab- und Neu-Einbaus eines Nukleotids wandert der Einzelstrang-Bruch in Richtung 3′-Ende. Bis zu vier der als Substrat angebotenen Triphosphat-Arten sind wiederum teilweise oder gänzlich durch Kopplung eines Steroids über eine Brücke derivatisiert, so daß beim Neueinbau der Nukleotide dieses Steroid mit eingebaut wird.

In der "Tailing" Methode wird an 3′-OH-Enden von doppel-oder einzelsträngigen Desoxyribo- oder Ribonukleinsäure-Sonden mit dem Enzym Terminale Transferase in Anwesenheit mindestens einer Art der als Substrat dienenden Desoxyribonukleosidtriphosphate, Dideoxyribonukleosidtriphosphate oder Ribonukleosidtriphosphate mindestens eines dieser Nukleotide angehängt. Die verwendete Art der Triphosphate ist wiederum teilweise oder gänzlich durch Kopplung eines Steroids über eine Brücke derivatisiert, so daß bei dem Einbau der Nukleotide dieses Steroid mit eingebaut wird.

Bei der Phagen-RNA-Polymerase-katalysierten "Transkriptions"-Methode (J. Mol. Biol. 166 (1983) 477) wird während der Desoxyribonukleinsäure-abhängigen Ribonukleinsäure-Synthese die gebildete Ribonukleinsäure derivatisiert. Als Phagen-codierte RNA-Polymerasen werden z.B. Phage SP6-, T7- oder T3-codierte Enzyme verwendet. Für diese Methode werden doppelsträngige Desoxyribonukleinsäuren verwendet, die z.B. SP6-, T7- oder T3-Promotoren enthalten. Durch Zugabe von z.B. SP6-, T7- oder T3-RNA Polymerase und aller vier Arten der Ribonukleosid-Triphosphate wird, ausgehend vom homologen Promotor, der zur codogenen Desoxyribonukleinsäure komplementäre Ribonukleinsäure-Strang, das Transkript, gebildet. Dabei werden die als Substrat angebotenen Ribonukleosidtriphosphat-Arten eingebaut. Bis zu vier dieser Triphosphat-Arten sind teilweise oder gänzlich durch Kopplung eines Steroids über eine Brücke derivatisiert, so daß bei der Ribonukleinsäure-Synthese dieses Steroid mit eingebaut wird.

In der "photochemischen" Methode (Nucl. Acids Res. 13 (1985) 745-761) wird die Nukleinsäure-Sonde in Gegenwart von Photo-Digoxigenin (Fig. 2) mit sichtbarem Licht mit UV-Anteil bestrahlt. Unter Abspaltung von Stickstoff ($N_2$) entsteht ein Nitrenradikal, das kovalent an die Nukleinsäure bindet.

Für die "chemische" Methode werden im Rahmen der Oligo-Desoxyribonukleotidsynthesenach der Phosphit-Triester-Methode neben den geschützten Nukleosid-phosphoramiditen (dA/dG/dC/dT) geschützte, mit substituierbaren Aminofunktionen modifizierte Nukleosid-phosphoramidite (dA/dG/dC/dU) zum gezielten Einbau in den Oligo-Desoxyribonukleotid-Einzelstrang verwendet. Die Modifizierung von dC/dU geschieht bevorzugt in Position 5 des Pyrimidin-Ringes, die von dA/dG bevorzugt an Position 8 des Purinmoleküls.

Nach Abschluß der Synthesezyklen und Entfernung der Schutzgruppen resultieren einzelsträngige, an den Nukleobasen mit substituierbaren Aminofunktionen modifizierte Oligo-Desoxyribonukleotide, die mit geeigneten Haptenen markierbar sind. Solche Haptene sind Steroide, bevorzugt Digoxigenin, Digoxin, d.h. die Markierung geschieht durch Umsetzung des Oligo-Desoxyribonukleotides mit den entsprechenden aktivierten Estern, Amiden oder Ethern der Haptene, vorzugsweise ihren N-hydroxysuccinimidestern.

In einer bevorzugten Ausführungsform der Erfindung wird eine Nukleinsäure-Sonde verwendet, deren Hapten in die Nukleinsäure-Sonde enzymatisch mit Hilfe von DNA-Polymerasen, RNA-Polymerasen, Reversen Transkriptasen oder Terminalen Transferasen und entsprechenden Hapten-modifizierten Desoxy- oder Ribonukleosidtriphosphat-Substraten eingebaut wurde.

In einer weiteren bevorzugten Ausführungsform der Erfindung wird eine Nukleinsäure-Sonde verwendet, deren Hapten in die Nukleinsäure-Sonde photochemisch mit Hilfe von Photo-Hapten eingebaut wurde und in einer dritten bevorzugten Ausführungsform wird eine Nukleinsäure-Sonde verwendet, deren Hapten in die Nukleinsäure-Sonde chemisch im Rahmen der Oligo-Desoxyribonukleotid-Synthese durch Einbau geschützter, mit substituierbaren Aminofunktionen modifizierter Nukleosidphosphoamidite und - nach Entfernung der Schutzgruppen - durch Umsetzung des modifizierten Oligo-Desoxyribonukleotids mit aktivierten Estern der Haptene eingebaut wurde.

Die über eine der beschriebenen Methoden hergestellte, mit einem Steroid derivatisierte Nukleinsäure-Sonde wird mit einer an einen Träger gebundenen denaturierten DNA oder RNA in Kontakt gebracht und hierbei die Temperatur, Ionenstärken, pH-Wert und sonstige Pufferbedingungen - abhängig von der Länge der Nukleinsäureprobe und der daraus resultierenden Schmelztemperatur des zu erwartenden Hybrids - so gewählt, daß die markierte DNA oder RNA an homologe DNA oder RNA binden kann (Hybridisierung) (J. Mol. Biol. 98 (1975) 503; Proc. Natl. Acad. Sci. USA 76 (1979) 3683). Als Träger geeignet sind Membranen oder Trägermaterialien auf Basis Nitrozellulose (z.B. Schleicher und Schüll BA85; Amersham Hybond C), verstärkte oder gebundene pulverförmige Nitrocellulose oder mit verschiedenen funktionellen Gruppen (z.B. Nitro-) derivatisierte Nylonmembranen (z.B. Schleicher und Schüll Nytran, NEN Gene Screen, Amersham Hybond N, Pall Biodyne).

Die Detektion von hybridisierter DNA oder RNA erfolgt dann dadurch, daß der Träger nach gründlichem Waschen und Absättigung zur Verhinderung unspezifischer Bindungen mit einem Antikörper oder Antikörperfragment inkubiert wird. Der Antikörper oder das Antikörperfragment ist gegen das in die Nukleinsäure-Sonde bei der Derivatisierung inkorporierte Steroidhapten gerichtet. Er trägt konjugiert eine Markierung. Nach der Antikörper-Inkubation wird nochmals gewaschen, um nur spezifisch gebundene Antikörper-Konjugate nachzuweisen. Die Bestimmung erfolgt sodann über die Markierung des Antikörpers oder Antikörperfragments nach an sich bekannten Methoden.

Die Länge der Brücke, über die das Hapten an die Nukleinsäure-Probe gebunden ist, kann zwischen 4 und 32 Atome betragen. Die Brücke ist hierbei aus Molekülen aufgebaut, die die Atome C, O, S oder/und N enthalten. Eine größere Kettenlänge ist zwar möglich, aber nicht mehr sinnvoll, da mit einem Sensitivitätsverlust zu rechnen ist.

In einer bevorzugten Ausführungsform der Erfindung ist das Hapten über eine Brücke von 11 bis 16 Atomen Länge an die Probe gebunden. Bevorzugt enthält die Brücke neben hydrophoben auch hydrophile Gruppierungen.

In einer bevorzugten Ausführungsform ist die Brücke linear. In einer weiteren Ausführungsform besteht die Brücke aus einer verzweigten Kette und trägt an mindestens einem der Kettenenden ein Haptenmolekül. Durch das Vorhandensein mehrerer Haptenmoleküle an den Kettenenden einer verzweigtkettigen Brücke kann die Nachweissensitivität zusätzlich verstärkt werden.

Die Verbindung des Steroid-Haptens mit der Brücke ist vorzugsweise eine Ester-, Amid- oder Etherbindung.

Die Bindung des Steroid-Haptens über die Brücke an die Nukleinsäureprobe ist sowohl über eine endständige oder nicht endständige Phosphatgruppe, als auch über einen Zuckerrest oder eine Base der Nukleinsäure-Probe möglich. Die Bindung des Haptens über die Brücke muß jedoch so erfolgen, daß nicht die Wasserstoffbrückenbildung zwischen den beiden komplementären Nukleinsäuresträngen beeinträchtigt wird.

Bevorzugt ist das Hapten über die Brücke an eine Base oder den Ribose-Anteil der Nukleinsäure-Probe gebunden, wobei das Hapten besonders bevorzugt über die Brücke an die $C_5$-Position von Uracil oder Cytosin, die $C_8$-Position von Adenin oder Guanin oder an die 2′-Position der Ribose gebunden ist.

Die Markierung des Anti-Hapten-Antikörpers oder des Antikörperfragments erfolgt in an sich bekannter Weise. Geeignet sind z. B. Enzymmarkierung, radioaktive Markierung, (Bio-)Lumineszenzmarkierung oder Fluoreszenzmarkierung. Bevorzugt wird jedoch eine Enzymmarkierung mit Enzymen wie Alkalische Phosphatase, Peroxidase oder $\beta$-Galactosidase verwendet. Besonders bevorzugt wird als Markierungsenzym Alkalische Phosphatase verwendet. Die Bestimmung der Alkalischen Phosphatase erfolgt über Leukosysteme, insbesondere über Indigoide Systems als oxydierbare Verbindungen (siehe EP 228 663). Als Oxydationsmittel dienen Tetrazoliumsalze. Bei dem Markierungsenzym Alkalische Phosphatase wird als Redoxsystem bevorzugt X-Phosphat/Nitroblau-Tetrazolium eingesetzt (F.P. Altmann, Tetrazolium Salts and Formazans, Progr. Histochem. Cytochem. Vol. 913 (1976), Gustav Fischer Verlag, Stuttgart, S. 1). X-Phosphat ist hierbei ein Trivialname für 5-Brom-4-Chlor-3-Indolylphosphat, und Nitroblau-Tetrazolium steht für die Verbindung 3,3′-(3,3′-Dimethoxy-4,4′-Biphenylen)-bis-5-Phenyl-2-(4-Nitrophenyl)-Tetrazoliumchlorid (F.P. Altmann, Tetrazolium Salts and Formazans, Progr. Histochem. Cytochem. Vol. 913 (1976), Gustav Fischer Verlag, Stuttgart, pp. 1). Alkalische Phosphatase spaltet das chromogene Substrat, in diesem Fall X-Phosphat, das durch die Abspaltung des Phosphats und Oxidation ein blaues, schwerlösliches Dimer bildet, wobei gleichzeitig die Tetrazolium-Verbindung zu einem ebenfalls blauen, schwerlöslichen Formazan reduziert wird. Diese Redoxreaktion ist in Fig. 3 dargestellt.

Der Nachweis der anderen geeigneten Markierungssysteme ((Bio)-Lumineszenzmarkierung, Fluoreszenzmarkierung, radioaktive Markierung) wird nach an sich bekannten Methoden durchgeführt.

Das erfindungsgemäße Verfahren ist zur weiteren Veranschaulichung in Fig. 4 schematisch unter Verwendung von über eine Brücke von 11 Atomen an dUTP-gebundenem Digoxigenin als Hapten dargestellt.

Das erfindungsgemäße Verfahren kann besonders vorteilhaft angewendet werden zur:
in situ-Hybridisierung mit fixierten ganzen Zellen, mit fixierten Gewebsabstrichen und isolierten Chromosomen (Metaphasen-Chromosomen)
Colony-Hybridisierung (Zellen) und plaque-Hybridisierung (Phagen und Viren)
Northern-Hybridisierung (RNA-Nachweis)
Serum-Analytik (Nachweis von Virus- und bakteriellen Infektionen in Serum, Zelltyp-Analyse von Zellen im Serum; z.B. durch slot-blot-Analyse).

In einer weiteren bevorzugten Ausführungsform wird vor der Durchführung des erfindungsgemäßen Bestimmungsverfahrens eine Amplifikation der sample, wie in EP-A 0200362 beschrieben, durchgeführt.

Ein weiterer Gegenstand der Erfindung ist ein Verfahren zum Nachweis von Nucleinsäuren definierter Sequenz (sample), welches dadurch gekennzeichnet ist, daß man die sample, nach Denaturierung, mit mindestens zwei Oligonucleotiden (primer), von welchen ein erstes in seiner Sequenz komplementär zu einer Teilsequenz eines Stranges der nachzuweisenden Nucleinsäure und ein weiteres identisch mit einer anderen Teilsequenz desselben Stranges der nachzuweisenden Nucleinsäure ist, behandelt (Hybridisierung), mit Polymerase, vorzugsweise tag-DNA-Polymerase, mit Desoxyribonucleotiden und mit mindestens einem Desoxyribonucleotid, das chemisch gebunden als Hapten ein Steroid enthält, welches an mindestens eine Position des Desoxyribonucleotids, die nicht an der Wasserstoffbrückenbindung beteiligt ist, über eine Brücke von mindestens 4 Atomen Länge gebunden ist, behandelt (Polymerisierung) und anschließend den Zyklus, bestehend aus Denaturierung, Hybridisierung und Polymerisierung mindestens einmal wiederholt, wobei eine zur sample komplementäre Nucleinsäure entsteht, welche markiert ist und die so entstandene markierte Nucleinsäure über einen seinerseits markierten Anti-Hapten-Antikörper nachweist.
In einer bevorzugten Ausführungsform werden die Desoxyribonucleotide, die markierten Desoxyribonucleotide und die primer bereits vor der Denaturierungsreaktion zugesetzt.

In einer weiteren bevorzugten Ausführungsform wird nach Ablauf der Zyklen eine zur sample komplementäre, an eine Festphase immobilisierte Nucleinsäure zugegeben, eine Hybridisierungsreaktion durchgeführt und nach Trennung von fester und flüssiger Phase die Markierung in der Festphase bestimmt.

Ein weiterer Gegenstand der Erfindung ist ein Verfahren zum Nachweis von Nucleinsäuren definierter Sequenz (sample), welches dadurch gekennzeichnet ist, daß man die sample nach Denaturierung mit mindestens zwei Oligonucleotiden (primer), von welchen ein erstes in seiner Sequenz komplementär zu einer Teilsequenz eines Stranges der sample und ein weiteres identisch mit einer anderen Teilsequenz desselben Stranges der nachzuweisenden Nucleinsäure ist, behandelt (Hybridisierung), mit Desoxyribonucleotiden, mit Polymerase, vorzugsweise tag-DNA-Polymerase, behandelt (Polymerisierung), anschließend den Zyklus, bestehend aus Denaturierung, Hybridisierung und Polymerisierung mindestens einmal wiederholt und abschließend einen Zyklus in Gegenwart von mindestens einem Desoxyribonucleotid, welches chemisch gebunden als Hapten ein Steroid enthält, welches an eine Position des Desoxyribonucleotids, die nicht an der Wasserstoffbrückenbindung beteiligt ist, über eine Brücke von mindestens 4 Atomen Länge gebunden ist, wobei eine zur sample komplementäre Nucleinsäure entsteht, die markiert ist und die so entstandene markierte Nucleinsäure über einen seinerseits markierten Anti-Hapten-Antikörper nachweist.

In einer weiteren bevorzugten Ausführungsform wird nach Ablauf der Zyklen eine zur sample komplementäre, an eine Festphase immobilisierte Nucleinsäure zugegeben, eine Hybridisierungsreaktion durchgeführt und nach Trennung von fester und flüssiger Phase die Markierung in der Festphase bestimmt.

Die weiteren Bedingungen für diese Verfahren sind beispielsweise in der EP-A 0200362 beschrieben und unter dem Namen Polymerase chain reaction (PCR) bekannt.

Die folgenden Beispiele erläutern in Verbindung mit den Abbildungen die Erfindung näher.

Fig. 1 zeigt ein über eine Brücke von 11 Atomen Länge mit einem Digoxigenin-Molekül markiertes Desoxyuridintriphosphat;

Fig. 2 zeigt Photodigoxigenin;

Fig. 3 zeigt die Farbreaktionen beim Nachweis des Enzyms Alkalische Phosphatase über das Redoxsystem X-Phosphat/Nitroblau Tetrazolium;

Fig. 4 zeigt schematisch eine bevorzugte Ausführungsform des erfindungsgemäßen DNA-Nachweises;

Fig. 5 deutet die Herstellung eines RNA-Transkripts nach der SP6- bzw. T7-RNA-Polymerase katalysierten Transkriptionsmethode mit den Plasmiden pSPT18 an;

Fig. 6 zeigt die unterschiedlichen DNA-Bereiche der Plasmide pSPT18;
Fig. 7 zeigt ein Syntheseschema für die Herstellung von Photodigoxigenin;
Fig. 8 zeigt einen Vergleich der Sensitivitäten des erfindungsgemäßen Nachweises mit Digoxigenin als Hapten gegenüber dem Nachweis über Biotin/Streptavidin im Nachweis von Plazenta-DNA nach Southern-Blotting und Hybridisierung;
Fig. 9 zeigt einen Vergleich der Sensitivitäten einer erfindungsgemäßen enzymatischen Markierung (A) mit einer bekannten chemischen Markierung (B) im DNA-Nachweis; und
Fig. 10 zeigt die DNA-Sequenz (A) und Restriktionskante (B) von pSPT18.

**Beispiel 1**

Digoxigenin-O-succinyl-[5-(amidoallyl)-2′-desoxy-uridin-5′-Triphosphat]-Tetralithiumsalz

(Dig-4-dUTP)

$C_{39}H_{52}O_{21}N_3P_3Li_4$ MG 1019,5

200 mg Digoxigenin-O-succinyl-N-hydroxysuccinimidester (0,34 mMol) werden in 7 ml Dimethylformamid gelöst und einer Lösung von 186 mg 5-Allylamino-2′-desoxy-uridin-5′-triphosphat-Tetralithiumsalz (0,34 mMol) in 6 ml $H_2O$ zugefügt. Man gibt zu dem Reaktionsgemisch 62 ml 0,1 m Na-boratpuffer, pH 8,5 und rührt ca. 15 Stunden bei Raumtemperatur über Nacht.

Nach dieser Zeit beobachtet man papierelektrophoretisch (0,05 m-Citratpuffer, pH 5,0) unter UV-Licht neben unumgesetztem 5-Allylamino-dUTP einen etwas tiefer laufenderen Fleck des gewünschten Produktes.

Die Aufreinigung geschieht wie unter Beispiel 9 beschrieben.

Ausbeute: 130 mg = 37% der Th.

UV-Spektrum (Phosphatpuffer pH 7,0): Maxima: 220 nm, 290 nm

**Beispiel 2**

Digoxigenin-O-succinyl-ε-amidocapronsäure

$C_{33}H_{49}O_9N$ MG: 603,8

In einem 250 ml-Rundkolben werden 5 g Digoxigenin-O-succinyl-N-hydroxysuccinimidester (8,5 mMol) in 150 ml Dimethylformamid (DMF) gelöst und dazu eine Suspension von 1,12 g 6-Aminocapronsäure (8,5 mMol) und 1,2 ml Triethylamin in 20 ml DMF gegeben. Man rührt über Nacht bei Raumtemperatur magnetisch, wobei allmählich eine homogene Lösung entsteht. Nach dieser Zeit ist die Umsetzung laut Dünnschichtchromatographie (Kieselgel; Essigsäureethylester/Petrolether/Ethanol 1:1:1,

Detektion: Besprühen mit einer Mischung von 10 ml Eisessig + 0,2 ml konz. $H_2SO_4$ + 0,1 ml Anisaldehyd und Erhitzen auf 120 °C bis zum Erscheinen blauschwarzer Flecke; $R_f$ ca. 0,7; $R_f$ Digoxigenin-OSu-ester ca. 0,85) praktisch vollständig.

Man destilliert DMF im Hochvakuum restlos ab und löst das verbleibende Öl in 50 ml $H_2O$ unter Zugabe von konz. Ammoniaklösung. Dann wird durch Zufügen von 225 ml wäßriger Zitronensäurelösung (100 g Zitronensäure/l) die "freie" Digoxigeninamidocapronsäure abgeschieden. Die harzig-zähe Masse wird durch Anreiben mit Wasser fest; man saugt ab, wäscht mehrfach mit $H_2O$ nach und trocknet letztlich über $P_2O_5$ im Ölpumpenvakuum.

Ausbeute: 3,45 g = 68% der Th.

**Beispiel 3**

Digoxigenin-O-succinyl-ε-amidocapronsäure-N-hydroxysuccinimidester

$C_{37}H_{52}O_{11}N_2$ MG: 700,8

In einem 100 ml-Rundkolben werden 3,45 g Digoxigenin-O-succinyl-ε-amidocapronsäure (5,7 mMol) in 20 ml wasserfreiem Dimethylformamid (DMF) gelöst, und nacheinander mit 0,7 g N-Hydroxysuccinimid (6 mMol), sowie 1,3 g Dicyclohexylcarbodiimid (6,3 mMol) versetzt. Man rührt über Nacht bei Raumtemperatur, saugt anderntags vom abgeschiedenen Dicyclohexylharnstoff ab und zieht das DMF im Ölpumpenvakuum ab. Das zurückbleibende Öl wird in 20 ml Essigsäureethylester aufgenommen und in ca. 150 ml eiskalten (-20 °C) Petrolether eingerührt. Das ausgefallene, anfangs noch harzig-zähe Produkt reibt man mehrfach mit eiskaltem trockenem Petrolether bis zum Festwerden durch. Nach Trocknung über $P_2O_5$ im Vakuum erhält man
3,35 g = 84 % der Th.

| Elementaranalyse: | | |
|---|---|---|
| C ber.: 63,4%<br>C gef.: 63,1% | H ber.: 7,5%<br>H gef.: 7,7% | N ber.: 4,0 %<br>N gef.: 4,07%. |

**Beispiel 4**

Digoxigenin-O-succinyl-ε-amidocaproyl-[5-(amidoallyl)-2′-desoxy-uridin-5′-triphosphat]-Tetranatriumsalz

(Dig-11-dUTP)

$C_{45}H_{63}O_{22}N_4P_3Na_4$ MG: 1196,7

260 mg Digoxigenin-O-succinyl-ε-amidocapronsäure-N-hydroxysuccinimidester (0,37 mMol) werden in 7 ml DMF gelöst und zu einer Lösung von 200 mg 5-Allylamino-2′-desoxy-uridin-5′-triphosphat-Tetralithiumsalz (0,37 mMol) in 6 ml $H_2O$ gegeben. Man fügt dem Gemisch 62 ml 0,1 m-Natriumboratpuffer, pH 8,5 zu und rührt bei Raumtemperatur über Nacht (ca. 15 Stunden).

In der Papierelektrophorese (0,05 m-Citratpuffer, pH 5,0) beobachtet man im UV-Licht nach dieser Zeit neben etwas unumgesetztem Allylamino-dUTP einen etwas tiefer laufenderen Fleck der gewünschten Verbindung (alternativ: Dünnschichtchromatographie (DC) auf Kieselgel, Fließmittel Isobuttersäure/konz. Ammoniaklösung/$H_2O$ = 66:1:33, Detektion im UV oder Besprühen mit Anisaldehyd-Reagenz - siehe Beispiel 2 -; $R_f$-Werte: 5-Allylamino-dUTP 0,2; Dig-amidocapronsäure-OSu-ester 0,7; Dig-11-dUTP 0,45).

Zur Aufreinigung wird das Reaktionsgemisch im Ölpumpenvakuum bis zum festen Rückstand eingedampft, in 200 ml $H_2O$ aufgenommen und auf eine Ionenaustauschersäule (DEAE-Sephadex® A25, $HCO_3^-$-Form, Säulendimension 1,5 x 30 cm) gegeben. Nach Aufziehen wird kurz mit Wasser gewaschen, dann mit einem Gradient von je 1 l $H_2O$ auf 0,4 m TEAB (Triethylammoniumbicarbonat), pH 8 eluiert. Die reines Produkt enthaltenden Fraktionen werden vereinigt, im Vakuum konzentriert und durch mehrfaches Eindampfen mit Methanol von überschüssigem TEAB befreit (kein Geruch von freiem Triethylamin mehr!). Man nimmt den Kolbeninhalt in wenigen ml Wasser auf, passiert die Lösung über eine kurze Kationenaustauschersäule DOWEX® 50 WS8 (1 x 10 cm) in der $Na^+$-Form, wäscht die Säule bis zur ODE-Freiheit des Waschwassers (Messung im UV bei 240 nm) und dampft im Vakuum bis auf ca. 20 ml ein. Nach Lyophilisation werden 200 mg (45% der Th.) Dig-11-dUTP-$Na_4$ als weißes Pulver erhalten.
Analytik: $H_2O$-Bestimmung: 7,9%

| Elementaranalyse: (unter Berücksichtigung des $H_2O$-Gehaltes): | | | |
|---|---|---|---|
| C ber.: 41,8%<br>C gef.: 41,08% | H ber.: 5,3%<br>H gef.: 5,35% | N ber.: 4,3%<br>N gef.: 4,7% | P ber.: 7,2%<br>P gef.: 7,1%. |

UV-Spektrum (Phosphatpuffer pH 7,0): Maxima: 220 nm, 290 nm.

**Beispiel 5**

Digoxigenin-O-succinyl-γ-amidobuttersäure

$C_{31}H_{45}O_9N$ MG: 575,8

Die Verbindung wird durch Umsetzung von 3 g Digoxigenin-O-succinyl-N-hydroxysuccinimidester (5,1 mMol) mit 0,63 g 4-Aminobuttersäure (6,1 mMol) wie in Beispiel 1 für das Capronsäurederivat beschrieben, hergestellt. Nach erfolgter Umsetzung wird das Reaktionsgemisch im Vakuum eingedampft, der Rückstand in $H_2O$-Methanol (20%) gelöst und über eine Kationenaustauschersäule (DOWEX® 50 WX8) in der $H^+$-Form gegeben. Eluat und Waschwasser (pH ca. 4) werden eingedampft, der zurückbleibende schmierig-zähe Rückstand in n-Butanol gelöst und dreimal mit Wasser ausgeschüttelt. Die Butanol-Phase enthält das gewünschte Produkt und wird nach Abziehen des Butanols und dreimaliger Codestillation mit wasserfreiem DMF (Entfernung restlichen Wassers) direkt zur Weiterverarbeitung zum entsprechenden N-Hydroxy-succinimidester (Beispiel 6) eingesetzt.
Ausbeute: 2,94 g (Öl)

**Beispiel 6**

Digoxigenin-O-succinyl-γ-amidobuttersäure-N-hydroxysuccinimidester

$C_{35}H_{48}O_{11}N_2$ MG: 672,8

2,94 g Digoxigenin-O-succinyl-γ-amidobuttersäure (ca. 5,1 mMol) werden als Öl aus Beispiel 5 mit 0,62 g N-Hydroxysuccinimid (5,4 mMol) und 1,16 g Dicyclohexylcarbodiimid (5,6 mMol) in 20 ml wasserfreiem DMF wie unter Beispiel 3 beschrieben, umgesetzt und aufgearbeitet. Der resultierende Hydroxysuccinimidester wird als Öl - wie in Beispiel 7 beschrieben - mit ε-Aminocapronsäure umgesetzt.
Ausbeute: 3,46 g (Öl)

**Beispiel 7**

Digoxigenin-O-succinyl-γ-amidobutyryl-ε-amidocapronsäure

$C_{37}H_{56}O_{10}N_2$ MG: 689,0

In einem 250 ml-Rundkolben werden 0,8 g ε-Aminocapronsäure (6,2 mMol) und 0,75 ml Triethylamin in 12 ml DMF suspendiert und dazu eine Lösung von 3,46 g Digoxigenin-O-succinyl-γ-amidobuttersäure-N-hydroxysuccinimidester (5,1 mMol, Öl aus Beispiel 6) in 90 ml DMF gegeben. Man läßt über Nacht ca. 15 Stunden bei Raumtemperatur rühren, wonach eine nahezu homogene Lösung entstanden ist. Laut DC (Bedingungen siehe unter Beispiel 2) ist die Umsetzung fast quantitativ.

Die Aufarbeitung geschieht wie unter Beispiel 5 beschrieben (Überführung in die "freie" Carbonsäure durch DOWEX® 50-Chromatographie, Extraktion mit n-Butanol). Die Butanol-Phase enthält neben dem gesuchten Produkt noch etwas polareres und unpolareres Material und wird deswegen durch Chromatographie an Kieselgel 60 (Säule 40x3cm, Elutionsmittel Essigsäurethylester/Petrolether 50/75/Ethanol 1:1:1) gereinigt. Nach Vereinigen der sauren Fraktionen und Eindampfen erhält man als Öl
1,48 g = 42% der Th.

**Beispiel 8**

Digoxigenin-O-succinyl-γ-amidobutyryl-ε-amidocapronsäure-N-hydroxysuccinimid-ester

$C_{41}H_{59}O_{12}N_3$ MG: 785,8

0,2 g Digoxigenin-O-succinyl-γ-amidobutyryl-ε-amidocapronsäure (Öl aus Beispiel 7, ca. 0,29 mMol) werden mit 0,034 g N-hydroxysuccinimid (0,3 mMol) und 66 mg Dicyclohexylcarbodiimid (0,32 mMol) in 8 ml wasserfreiem DMF wie unter Beispiel 3 beschrieben, umgesetzt und aufgearbeitet. Der erhaltene ölige Rückstand ist auch durch mehrfaches Durchreiben mit kaltem Petrolether nicht festzubekommen und wurde daher nach Abziehen der Lösungsmittel direkt - wie in Beispiel 9 beschrieben - mit 5-Aminoallyl-dUTP zur Reaktion gebracht.
Ausbeute: 0,25 g (Öl)

**Beispiel 9**

Digoxigenin-O-succinyl-γ-amidobutyryl-ϵ-amidocaproyl-[5-amidoallyl)-2′-desoxy-uridin-5′-triphosphat]-Tetralithiumsalz

(Dig-16-dUTP)

$C_{49}H_{70}O_{23}N_5P_3Li_4$ MG 1217,7

250 mg Digoxigenin-O-succinyl-γ-amidobutyryl-ϵ-amidocapronsäure-N-hydroxy-succinimid-ester (Öl aus Beispiel 8, ca. 0,3 mMol) werden in 7 ml DMF gelöst und zu einer Lösung von 210 mg 5-Allylamino-2′-desoxy-uridin-5′-triphosphat-Tetralithiumsalz (0,38 mMol) in 6 ml $H_2O$ gegeben. Man gibt dem Reaktionsgemisch 62 ml 0,2 m Natriumboratpuffer, pH 8,5 zu und rührt ca. 15 Stunden über Nacht bei Raumtemperatur. Der Reaktionsablauf wird wie unter Beispiel 4 beschrieben, verfolgt.

Zur Aufreinigung wird der Ansatz im Ölpumpenvakuum bis zum festen Rückstand eingedampft, in ca. 200 ml $H_2O$ gelöst und auf eine Ionenaustauschersäule (DEAE-Sephadex® A-25, $Cl^-$-Form, Säulenabmessung 1,5 x 30 cm) gegeben. Nach Waschen mit $H_2O$ wird mit einem linearen Gradienten von 2 l $H_2O$ auf 2 l 0,3 m-LiCl eluiert. Die das reine Produkt enthaltenden Fraktionen werden vereinigt, im Vakuum so weit konzentriert, bis kein $H_2O$ mehr übergeht und das Konzentrat anschließend in einem Aceton-Ethanol-Gemisch (3:1) durch Einrühren gefällt. Man zentrifugiert vom Überstand ab, wäscht bis zur $Cl^-$-Freiheit mit Ethanol und trocknet mit Vakuum über $P_2O_5$/KOH.

Ausbeute: 250 mg = 68 % der Th.

Analytik: $H_2O$-Bestimmung: 6,3%.

| Elementaranalyse (unter Berücksichtigung des $H_2O$-Gehaltes): | | | |
|---|---|---|---|
| C ber.: 45,5%<br>C gef.: 45,1% | H ber.: 5,7%<br>H gef.: 5,6% | N ber.: 5,4%<br>N gef.: 5,6% | P ber.: 7,2%<br>P gef.: 7,0% |

UV-Spektrum (Phosphatpuffer pH 7,0):
Maxima: 220 nm (Schulter)
289 nm

**Beispiel 10**

Digoxigenin-O-succinyl-ϵ-amidocaproyl-[5-(amidoallyl)-uridin-5′-triphosphate]-Tetralithiumsalz

(Dig-11-UTP)

$C_{45}H_{63}O_{23}N_4P_3Li_4$ MG: 1148,5

Die Verbindung wird durch Umsetzung von 520 mg Digoxigenin-O-succinyl-ϵ-amidocapronsäure-N-hydroxysuccinimidester (0,74 mMol) mit 416,5 mg 5-Allylamino-UTP-Tetralithiumsalz (0,74 mMol) analog Beispiel 4 hergestellt. Die Ionenaustauscherchromatographie erfolgt jedoch in Abwandlung nach Beispiel 9 an DEAE-Sephadex®-A-25 in der $Cl^-$-Form.

Ausbeute: 560 mg = 66 % d. Th.

Analytik: $H_2O$-Bestimmung: 8,1%

| Elementaranalyse (unter Berücksichtigung des $H_2O$-Gehaltes): | | | |
|---|---|---|---|
| C ber.: 43,5%<br>C gef.: 43,1% | H ber.: 5,47%<br>H gef.: 5,3% | N ber.: 4,5%<br>N gef.: 4,5% | P ber.: 7,47%<br>P gef.: 7,35% |

UV-Spektrum (Phosphatpuffer pH 7,0):
entspricht Dig-11-dUTP

**Beispiel 11**

Digoxigenin-O-succinyl-γ-amidobutyryl-ε-amidocaproyl-[5-(amidoallyl)-uridin-5′-triphosphat]-Tetralithiumsalz

(Dig-16-dUTP)

$C_{49}H_{70}O_{24}N_5P_3Li_4$ MG: 1233,7

Die Verbindung wird durch Umsetzung von 250 mg Digoxigenin-O-succinyl-γ-amidobutyryl-ε-amidocapronsäure-N-hydroxysuccinimidester (0,3 mMol, nach Beispiel 8 erhalten) mit 214 mg 5-Allylamino-UTP-$Li_4$ (0,38 mMol) analog Beispiel 9 hergestellt.
Ausbeute: 218 mg = 59 % d. Th.
Analytik: $H_2O$-Bestimmung = 7,2%

| Elementaranalyse (unter Berücksichtigung des $H_2O$-Wertes): | | | |
|---|---|---|---|
| C ber.: 44,45%<br>C gef.: 44,3 % | H ber.: 5,67%<br>H gef.: 5,5 % | N ber.: 5,3%<br>N ber.: 5,3% | P ber.: 7,0%<br>P gef.: 7,1% |

UV-Spektrum (Phosphatpuffer pH 7,0):
entspricht Dig-16-dUTP

**Beispiel 12**

Herstellung von N-Azidobenzoyl-1,8-diamino-3,6-dioxaoctan

5,20 g (20 mmol) Azidobenzoesäure-N-hydroxysuccinimidester (Fa. Pierce, D-6054 Rodgau 1) werden in wasserfreiem Essigester gelöst und mit 29,3 ml (200 mmol) 1,8-Diamino-3,6-dioxaoctan versetzt. Die Reaktionsmischung läßt man 20 Stunden bei 20 °C im Dunkeln rühren. Das Lösungsmittel wird im Vakuum entfernt und der ölige Rückstand in 300 ml Wasser gelöst. Das Produkt wird mit 2 l Toluol in einem Perforator aus der wäßrigen Phase extrahiert, wobei man die Apparatur mit Aluminiumfolie umwickelt. Die Extraktion ist nach ca. 16 Stunden beendet. Die organische Phase wird am Vakuum vom Lösungsmittel befreit und das Produkt durch präparative Säulenchromatographie an Kieselgel (Säule 80 x 10 cm, Eluent: Chloroform/Methanol/konz. Ammoniaklösung 65:30:5) aufgereinigt und nach Abdampfen des Lösungsmittels im Hochvakuum getrocknet.
Ausbeute: 3,2 g (55%); farbloses, zähes Öl.

**Beispiel 13**

Herstellung von Digoxigenin-3-hemisuccinat[N′-(4-azidobenzoyl)]-8-amino-3,6-dioxaoctylamid (Photodigoxigenin)

2,93 g (10 mmol) des Produktes aus Beispiel 12 werden in 200 ml wasserfreiem Dioxan gelöst und mit 5,87 g (10 mmol) Digoxigenin-3-hemisuccinat-N-hydroxysuccinimidester (Herstellung analog: G. C. Oliver, Jr. Brent, M. Parker, D.L. Brasfield und Ch.W. Parker; J. clin. Invest. 47 (1986), 1035) versetzt. Man läßt 20 Stunden bei Raumtemperatur rühren, entfernt das Lösungsmittel im Vakuum und trennt das entstandene Produkt durch präparative Mitteldruck-Flüssig-Chromatographie ab Säulenvolumen: 1640 ml, Labochrom Reversed-Phase-Silica HD-SIL-18-30-60, Eluent Methanol/Wasser 7:3 + 1 % Eisessig). Nach Sammlung der entsprechenden Fraktionen wird das Lösungsmittel im Vakuum entfernt und der ölige Rückstand in Dioxan Gelöst. Nach Lyophilisation und Waschen mit 100 ml Diisopropylether wird das Produkt Digoxigenin-3-hemisuccinat[N′-(4-azidobenzoyl)]-8-amino-3,6-dioxaoctylamid als farbloser, leicht klebriger Feststoff erhalten, der im Hochvakuum getrocknet wird.
Ausbeute: 4,9 g (64%).
IR (Aceton): = 2150, 1728, 1639 $cm^{-1}$.

**Beispiel 14**

Markierung einer Nukleinsäure-Sonde nach der "Random Primed" Methode

1 µg der zu markierenden linearen DNA wurde in einem Volumen von 10 µl durch 5minütiges Kochen und anschliessendes Abschrecken auf Eis denaturiert. Danach wurden in einem Reaktionsgefäß je 1 µl einer 2 mmol/l Lösung von Desoxyadenosintriphosphat, Desoxycytidintriphosphat und Desoxyguanosintriphosphat und die denaturierte DNA vereinigt. Dazu wurde 1 µl einer Lösung, die 1,4 mmol/l Desoxythymidintriphosphat und 0,6 mmol/l, wie in Beispiel 4 hergestelltes Digoxigenin-11-dUTP enthält zugesetzt. Danach wurden 2 µl eines sogenannten Reaktionsgemisches zugegeben. Das Reaktionsgemisch enthielt als wichtigste Komponente das sogenannte "Random-Hexanucleotid". Es handelt sich dabei um chemisch synthetisierte Oligonukleotide von 6 Basen Länge, wobei bei der Synthese in jedem Schritt alle 4 Nukleotide (A, C, G und T) angeboten werden und Somit ein Gemisch aller möglichen Oligonukleotid-Sequenzen entsteht. Die chemische Zusammensetzung des Reaktionsgemisches war: Tris-HCl, 0,5 mol/l; $MgCl_2$, 0,1 mol/l; Dithioeritrit, 1 mmol/l; Rinderserum-Albumin, molekularbiologische Qualität, 2 mg/ml; Random Hexanucleotid, 3,125 mg/ml; pH 7,2 (20 °C); zuletzt wurde 1 µl Klenow Polymerase, entsprechend 2 Einheiten, zugegeben und die Mischung mit sterilem Wasser auf 20 µl Endvolumen gebracht und 1 Stunde bei 37 °C inkubiert.

Danach wurde die Reaktion durch Zugabe von 2 µl 0,2 mol/l EDTA (Ethylendinitrilo-Tetraessigsäure), pH 8,0 gestoppt und uneingebaute Desoxyribonukleosidtrisphosphate wurden durch eine Ethanolfällung abgetrennt. Hierzu wurden dem Inkubationsansatz 2 µl LiCl, 4 mol/l, und 60 µl auf -20 °C vorgekühltes absolutes Ethanol zugegeben, gemischt und der Fällungsansatz bei -70 °C 30 Minuten lang oder bei -20 °C über Nacht inkubiert. Anschließend wurde bei 12000 x g (Erdbeschleunigung) 10 Minuten lang zentrifugiert, der Überstand abdekantiert, der Niederschlag kurz mitkaltem 70 %igem Ethanol gewaschen und zuletzt im Vakuum getrocknet. Die markierte gereinigte DNA wurde schließlich in 50 µl TE-Puffer (Tris-HCl, 10 mmol/l; EDTA, 1 mmol/l; pH 8,0) resuspendiert.

**Beispiel 15**

Markierung einer Nukleinsäure-Sonde nach der "Nick Translations" Methode

1 µg der zu markierenden DNA wurde gemischt mit je 1 µl einer 0,4 mmol/l Lösung von Desoxyadenosintriphosphat, Desoxycytidintriphosphat und Desoxyguanosintriphosphat.

Es wurde dann 1 µl einer Lösung, die 0,35 mmol/l Desoxythymidintriphosphat und 0,15 mmol/l wie in Beispiel 4 hergestelltes Dig-11-dUTP enthielt, zugesetzt. Desweiteren wurden 2 µl einer 10-fach konzentrierten Puffer-Lösung
(Tris-HCl, 0,5 mol/l; $MgCl_2$, 0,1 mol/l; Dithioerytrit, 1 mmol/l; pH 7,5) sowie 2 µl einer Enzym-Lösung, enthaltend 2 Einheiten DNA Polymerase I und 1.6 mEinheiten DNAseI zugesetzt, mit sterilem zweifach destilliertem Wasser auf ein Endvolumen von 20 µl aufgefüllt und der Ansatz 60 Minuten bei 14 °C inkubiert.

Die Reaktion wurde wie im Beispiel 13 mit EDTA gestoppt und die markierte DNA wie oben beschrieben gereinigt und gelöst.

**Beispiel 16**

Markierung einer Nukleinsäure-Sonde mit der "Tailing" Methode

Bei der 3′-Endmarkierung mit Hilfe des Enzyms Terminal Transferase wurde ein Digoxigenin-11-Didesoxyuridintriphosphat eingesetzt. Dieses wurde, wie im Beispiel 4 beschrieben, synthetisiert, mit der Ausnahme, daß als Ausgangssubstanz 5-Allylamino-2′,3′-didesoxy-uridin-5′-triphosphat-Tetralithiumsalz anstelle des 2′-Desoxyuridinsalzes eingesetzt wurde. Die Markierungsreaktion wurde wie folgt durchgeführt:
In einem Reaktionsgefäß wurden gemischt:
25 µl Markierungspuffer (Kalium-Kakodylat, 200 mmol/l; Tris-HCl, 50 mmol/l; Rinderserumalbumin, 0,4 mg/ml; pH 7,2), 5 µl 25 mmol/l $CoCl_2$-Lösung, 1,5 µg HaeII-gespaltene pBR322-DNA und 5 µl entsprechend 25 Einheiten Terminal Transferase. Das Volumen wirde mit sterilem zweifach destilliertem Wasser auf 48 µl gebracht und schließlich 2 µl einer 0,1 mmol/l Lösung von Digoxigenin-11-ddUTP zugegeben.

Nach Mischen und Zentrifugation wurde die Reaktion bei 37 °C für eine Stunde inkubiert.

Das Abstoppen der Reaktion und die Abtrennung uneingebauter Nukleotide erfolgte wie oben im Beispiel 14 beschrieben.

**Beispiel 17**

Markierung einer Nukleinsäure-Sonde mit der "Transkriptions" Methode

Das Prinzip der Reaktion ist der Fig. 5 zu entnehmen. Die zur Markierung verwendete DNA wurde in den Transkriptionsvektor pSPT18 (Fig. 6 und Fig. 10) insertiert. Der Vektor enthält einen Promotor für die SP6 und einen Promotor für die T7 RNA Polymerase. Die DNA wurde vor der Markierungsreaktion an einer Stelle außerhalb der insertierten DNA-Sequenz und der Promotoren, sowie den die Promotoren und die DNA-Sequenz verbindenden Sequenzen linearisiert.

1 µg der linearisierten Substrat-DNA wurde in einem Reaktionsgefäß je 1 µl einer 10 mmol/l Lösung von Adenosintriphosphat, Cytidintriphosphat und Guanosintriphosphat zugesetzt. Dazu wurde 1 µl einer Lösung, die 6,5 mmol/l Uridintriphosphat und 3,5 mmol/l Digoxigenin-11-UTP enthält, zugegeben. Digoxigenin-11-UTP wird im Beispiel 10, ähnlich wie im Beispiel 4 Digoxigenin-11-dUTP hergestellt, als Ausgangssubstanz wird lediglich anstelle des Allylamino-Desoxyuridinsalzes das Allylamino-Uridinsalz eingesetzt.

Weiterhin wurden dem Ansatz 2 µl eines 10fach konzentrierten Puffers (Tris-HCl, 0,4 mol/l; $MgCl_2$, 60 mmol/l; Dithiothreitol, 50 mmol/l; Spermidin, 40 mmol/l; pH 7,2) zugegeben, das Volumen auf 19 µl mit sterilem bidestilliertem Wasser aufgefüllt und schließlich die Reaktion durch Zugabe von 1 µl, entsprechend 10 Einheiten der RNA Polymerase (SP6 bzw. T7) gestartet.

Nach kurzer Zentrifugation wurde der Ansatz bei 37° C für eine Stunde inkubiert.

Die Substrat-DNA wurde anschließend durch Zusatz von 1 µl DNAseI, RNAse-frei, entsprechend 10 Einheiten, 15 Minuten bei 37° C abgebaut.

Die Reaktion wurde durch Zugabe von 2 µl 0,2 mol/l EDTA, pH 8,0 gestoppt. Die erhaltene Digoxigenin-markierte RNA-Probe wurde durch Extraktion mit 1 Volumen Phenol und durch anschließende Ethanol-Präzipitation von Proteinen und Nukleotiden gereinigt und schließlich in sterilem Puffer (Tris-HCl, 10 mmol/l; EDTA, 1 mmol/l; pH 8,0) gelöst.

**Beispiel 18**

Markierung einer Nukleinsäure-Sonde mit der "photochemischen" Methode

Die gereinigte DNA-Lösung (keine organischen Puffer) vorzugsweise in einer Konzentration von 0,5 bis 1,0 µg/µl wurde in einem Eppendorf Reaktionsgefäß im Halbdunkeln mit demselben Volumen einer 1 mg/ml Photodigoxigenin-Lösung (Beispiel 13) vermischt. Das Gemisch wurde unter Eiskühlung mit einer Philips HPLR 400 W Lampe in 10 cm Abstand 10 bis 30 Minuten lang von oben durch die Gefäßöffnung bestrahlt. Das Reaktionsgemisch mußte dabei kalt bleiben.

Nach der Reaktion wurde mit 0,1 M Tris-HCl, pH 9,0, 1,0 mM EDTA auf 100 µl aufgefüllt.

Die Lösung wurde mit 100 µl Butan-2-ol ausgeschüttelt, kurz zentrifugiert und die obere Butanolphase entfernt.

Die Butan-2-ol Extraktion wurde einmal wiederholt, die wäßrige Phase sollte jetzt auf 30 bis 40 µl konzentriert sein.

Nach Zugabe von Träger (Carrier) DNA (nur bei kleinen DNA Mengen) wurde durch Zugabe von 5 µl 3 M Natriumacetat oder einem entsprechenden Salz (z.B. 4 M LiCl) und 100 µl Ethanol bei -20° C über Nacht gefällt.

Nach Zentrifugation bei 4° C für 15 Minuten in einer Eppendorfzentrifuge wurde das Pellet mit kaltem 70% Ethanol gewaschen, getrocknet und in 0,1 mM EDTA gelöst.

**Beispiel 19**

Hybridisierung mit einer markierten DNA-Sonde

Zur Hybridisierung wurde ein Nitrocellulosefilter (Schleicher & Schüll, BA 85) mit darauf fixierter DNA verwendet. Zur Vorhybridisierung wurde der Filter zunächst 1 Stunde in einer Lösung, bestehend aus NaCl, 0,75 mol/l Na-Citrat, 75 mmol/l; Casein, 0,5 % (w/v); Laurylsarcosin, 0,1 % (v/v); Natriumdodecylsulfat, 0,02 % (w/v); pH 7,0 (20° C) bei 65° C inkubiert. Anschließend wurde die Lösung durch eine frische Lösung der selben Zusammensetzung ersetzt, der zusätzlich 100 ng/ml der wie in Beispiel 14 bis 16 oder 18 hergestellten markierten, frisch denaturierten DNA zugegeben wurden. Es erfolgte eine erneute Inkubation bei 65 °C für 14 Stunden. Danach wurde unspezifisch gebundene DNA durch zwei Waschschritte entfernt und zwar zuerst mit einer Lösung von NaCl, 0,3 mol/l; Na-Citrat, 30 mmol/l; Na-Dodecylsulfat, 0,1 % (w/v); pH 7,0 für 2 x 5 Minuten bei Raumtemperatur und anschließend mit einer Lösung von NaCl, 15 mmol/l; Na-Citrat, 1,5 mmol/l; Na-Dodecylsulfat, 0,1 % (w/v); pH 7,0 für 2 x 15 Minuten bei 65 °C.

**Beispiel 20**

Hybridisierung mit einer markierten RNA-Sonde

Hybridisierung und anschließende Waschungen wurden exakt wie im Beispiel 19, anhand der DNA-Probe geschildert, durchgeführt. Es wurde lediglich anstelle der im Beispiel geschilderten denaturierten markierten DNA-Probe eine wie im obigen Beispiel 17 markierte, frisch denaturierte RNA-Probe verwendet.

Die Konzentration der Digoxigenin-markierten RNA in der Hybridisierungslösung wurde so gewählt, daß pro ml Hybridisierungslösung die Transkripte von 100 ng Substrat-DNA eingesetzt wurden.

**Beispiel 21**

Detektion mit Hilfe des Nachweis-Systems Alkalische Phosphatase/X-Phosphat/Nitroblau-Tetrazolium

Der hybridisierte Filter aus Beispiel 19 oder 20 wurde zunächst 1 Minute in Tris-HCl, 0,1 mol/l, pH 7,5; NaCl, 0,15 mol/l gewaschen. Zur Blockierung unspezifischer Bindungsstellen der Membran wurde anschließend mit 0,5% (w/v) Casein in Tris-HCl 0,1 mol/l, pH 7,5; NaCl, 0,15 mol/l 40 Minuten lang unter leichtem Schwenken inkubiert.

Danach wurde die Antikörperbindungsreaktion durchgeführt. Dazu wurde die Membran mit Anti-Digoxigenin/Alkalische Phosphatase-Konjugat, 150 mEinheiten/ml in Tris-HCl, 0,1 mol/l; pH 7,5; NaCl, 0,15 mol/l 20 Minuten lang inkubiert. Unspezifisch gebundenes Protein wurde dann durch 2 x 15 minütiges Waschen mit dem selben Puffer entfernt. Danach wurde der Filter in Tris-HCl, 0,1 mol/l, pH 9,5; NaCl 0,1 mol/l; $MgCl_2$, 50 mmol/l 5 Minuten lang auf pH 9,5 umequilibriert, die Alkalische Phosphatase hat nämlich bei höheren pH-Werten ihr Aktivitätsoptimum. Der Nachweis gebundenen Antikörper-Konjugats und damit der hybridisierten markierten DNA erfolgte durch eine von der Alkalischen Phosphatase katalysierten Farbreaktion. Dazu wurde der Filter in Tris-HCl, 0,1 mol/l, pH 9,5; NaCl, 0,1 mol/l; $MgCl_2$, 50 mmol/l, wobei pro 10 ml Lösung 45 µl Nitroblau-Tetrazolium-Lösung (75 mg/ml in Dimethylformamid, 70 % (v/v)) und 35 µl einer X-Phosphatlösung (50 mg/ml 5-Bromo-4-Chloro-3-Indolylphosphat in Dimethylformamid) zugesetzt wurden, inkubiert.

Die Farbreaktion erfolgte unter weitgehendem Sauerstoffausschluß, indem man die Membran mit dieser Lösung in eine Folie (normale Koch- oder Gefrierbeutel) einschweißte und im Dunkeln aufbewahrte. Die Farbreaktion setzte sehr schnell ein, konnte aber an Intensität noch über mehrere Tage zunehmen. Eine Dokumentation des Ergebnisses wurde durch Photographie bzw. Photokopieren des feuchten Filters erhalten. Sodann wurde der Filter getrocknet und so aufbewahrt. Die Färbung blieb erhalten, verblaßte jedoch etwas. Sie ließ sich durch Anfeuchten des Filters wieder intensivieren.

**Beispiel 22**

Vergleich der Sensitivitäten und des Hintergrunds im DNA-Nachweis (Southern-Blot) bei Verwendung von a) nach Beispiel 14 mit Digoxigenin enzymatisch und b) mit Biotin enzymatisch markierter DNA.

I. Humane Plazenta-DNA wurde mit dem Restriktionsenzym EcoRI gespalten. Unterschiedliche Mengen davon wurden in einem Agarosegel elektrophoretisch aufgetrennt und anschließend nach der Methode von Southern (J. Mol. Biol. 98 (1975) 503) auf eine Nitrocellulose-Membran transferiert. Die DNA auf der Membran wurde durch 2 Stunden backen bei 80 °C im Vakuumschrank fixiert. Die Membranen wurden mit einem markierten DNA-Fragment, das der cDNA des humanen Gewebs-Plasminogenaktivators entspricht (Pennica D. et al, Nature 301 (1983) 214) hybridisiert. Das humane Gewebs-Plasminogenaktivatorgen ist im menschlichen Genom einmal vorhanden. Die cDNA dieses Gens hybridisiert mit den entsprechenden Banden des Genoms. Zum einen wurde das DNA-Fragment wie in Beispiel 14 mit Digoxigenin markiert, mit 100 ng/ml markierter DNA wie in Beispiel 19 hybridisiert und wie in Beispiel 21 beschrieben die Hybride detektiert. Zum anderen wurde das Fragment im analogen Verfahren mit Biotin markiert. Dabei wurde Biotin 16-dUTP (Brigati et al., Virology 126 (1983) 32-50) eingesetzt. Die DNA auf der Membran wurde mit 50 µg/ml markierter DNA hybridisiert. Ansonsten wurde nach Beispiel 19 verfahren. Die Detektion erfolgte wie in Beispiel 21 beschrieben, mit der Ausnahme, daß ein Streptavidin-Alkalische Phosphatase-Konjugat verwendet wurde. Im Vergleich zu in der Literatur beschriebenen Verfahren zum Biotinsystem zeigte das hier beschriebene System zur Biotinmarkierung ("Random primed"-Markierung, Mischung Biotin 16-dUTP/dTTP, Prähybridisierung mit der im Beispiel 19 angegebenen Lösung, Hybridisierung mit der angegebenen Konzentration an DNA, Blockierung mit der im Beispiel 21 angegebenen Lösung) geringeren Hintergrund und höhere Sensitivität. Dennoch ist bei der beschriebenen Verwendung von Digoxigenin anstelle von Biotin im optimierten System eine deutliche weitere Reduktion des Hintergrundes gegeben, wobei die Sensitivität erhalten bleibt. (Fig. 8)

**Beispiel 23**

Vergleich der Sensitivitäten im DNA-Nachweis (Southern-Blot) bei Verwendung von a) nach Beispiel 14 mit Digoxigenin enzymatisch und b) nach Beispiel 1 und 2 der europäischen Patentanmeldung 0173251 mit Digoxigenin chemisch markierter DNA.
pBR328 DNA wurde separat mit den Restriktionsenzymen BglI und HinfI gespalten. Die erhaltenen Fragmente wurden zu gleichen Teilen gemischt. DIe Mischung enthält danach 15 pBR328-Fragmente der Größe: 2176, 1766, 1230, 1033, 653, 517, 453, 394, 298 (2x), 234 (2x), 220 und 154 (2x) bp.

Unterschiedliche Mengen der Fragmente wurden dann in einem Agarosegel elektrophoretisch aufgetrennt und anschließend nach der Methode von Southern (J. Mol. Biol. 98 (1975) 503) auf eine Nitrocellulosemembran transferiert. Nach Fixieren der DNA-Fragmente durch Erhitzen der Membran im Vakuum auf 80 °C wurde diese mit 100 ng/ml nach Beispiel 14 mit Digoxigenin markierter DNA wie im Beispiel 19 beschrieben hybridisiert. Die Detektion der Hybride erfolgte exakt wie im Beispiel 21 beschrieben. Wie Fig. 9A in Verbindung mit Tabelle I zeigt, können Fragmente bis hinab zu einer Menge von 1 bis 0,1 pg nachgewiesen werden.

Als Vergleich wurde pBR328 DNA mit NciI gespalten. Es entstehen 10 Fragmente der Größe: 810, 724, 696, 597, 522, 507, 364, 351, 244 und 92 bp. Auch hier wurden unterschiedliche DNA-Mengen elektrophoresiert und anschließend nach Southern auf eine Nitrocellulosemembran transferiert. Die Membran wurde dann wie in Beispiel 19 beschrieben, hybridisiert. Es wurden allerdings hier 200 ng/ml wie in Beispiel 1 und 2 der EPA 0173251 markierte pBR322 DNA zur Hybridisierung eingesetzt. pBR328 DNA unterscheidet sich von der als Sonde eingesetzten pBR322 DNA lediglich durch das 810 bp große NciI-Fragment, welches pBR328 enthält, pBR322 dagegen nicht. Dieses wird also in der Hybridisierung nicht detektiert werden. Nach der Hybridisierung wurden die Hybride genauso wie im Beispiel 21 beschrieben detektiert. Wie Fig. 9B in Verbindung mit Tabelle II zeigt, können nach dieser Methode lediglich DNA-Fragmente bis zu einer Menge von 25 bis 2,5 pg nachgewiesen werden. Durch die enzymatische Markierung wird also im Vergleich zur chemischen Markierung eine um das Vielfache (Faktor 25) höhere Sensitivität der DNA-Detektion erreicht.

Tabelle I: Menge der DNA in den einzelnen Fragmenten einer Mischung von pBR 328-BglII-Fragmenten und

| Fragmentgröße | aufgetragene Menge (pg) DNA 125 | 25 | 5 | 1 |
|---|---|---|---|---|
| 2167 bp ≙ 22,08 % | 27,6* | 5,5* | 1,1* | 0,22* |
| 1766 bp ≙ 17,99 % | 22,5* | 4,5* | 0,9* | 0,18* |
| 1230 bp ≙ 12,53 % | 15,7* | 3,1* | 0,6* | 0,12 |
| 1033 bp ≙ 10,53 % | 13,2* | 2,6* | 0,5* | 0,1 |
| 653 bp ≙ 6,65 % | 8,3* | 1,66* | 0,3 | 0,07 |
| 517 bp ≙ 5,27 % | 6,6* | | | |
| 453 bp ≙ 4,62 % | 5,78* | | | |
| 394 bp ≙ 4,01 % | 5,0 | | | |

Menge in pg entsprechend der einzelnen Banden

* Banden, die auf dem Blot in Fig. 9A (feucht!) deutlich gefärbt sind.

Tabelle II

| pBR328, NciI-Spaltung: Menge der DNA in den einzelnen Fragmenten in Abhängigkeit von der Gesamt-DNA Menge pro Bahn. | | | | | |
|---|---|---|---|---|---|
| Fragment-Größe (bp) | Anteil, % der Gesamt-DNA | aufgetragene Menge DNA (pg) | | | |
| | | 100 | 200 | 1000 | 2000 |
| 810 | 16,5 | 16,5 | 33,0 | 165,1 | 330,1 |
| 724 | 14,8* | 14,8* | 29,5* | 147,5* | 295,1* |
| 696 | 14,2* | 14,2* | 28,4* | 141,8* | 283,7* |
| 597 | 12,2* | 12,2* | 24,3* | 121,7* | 243,3* |
| 522 | 10,6* | 10,6* | 21,3* | 106,4* | 212,8* |
| 507 | 10,3* | 10,3* | 20,7* | 103,3* | 206,6* |
| 364 | 7,4 | 7,4 | 14,8* | 74,2* | 148,4* |
| 351 | 7,2 | 7,2 | 14,3* | 71,5* | 143,1* |
| 244 | 5,0 | 5,0 | 9,9 | 49,7* | 99,4* |
| 92 | 1,9 | 1,9 | 3,7 | 18,7* | 37,5* |
| Summe: | | | | | |
| 4907 | 100 | | | | |
| Die auf der feuchten Membran des Blots von Fig. 9B noch gut sichtbaren Fragmente sind mit * markiert. | | | | | |

Beispiel 24

Nachweis von HPV-Sequenzen in fixierten HeLa-und SiHa-Zellinien durch in situ-Hybridisierung.

Mit dem erfindungsgemäßen Verfahren kann die in situ-Hybridisierung mit Träger-fixierten Zellen und Zellabstrichen durchgeführt werden.

Für den nichtradioaktiven HPV-Nachweis wurden zwei Zellinien verwendet:

SiHa-Zellen (2 - 3 Kopien HPV/Zelle) ATCC-Nr. HTB 35 (Biology 159 (1987) 389 - 398)

HeLa-Zellen (100 - 200 Kopien HPV/Zelle) ATCC-Nr. CCL 2

(Human Genetics 77 (1987) 251 - 255)

DNA-Probe:

HPV-DNA (Sequenz vgl. Progress med. Virol. 32 (1985) 15 ff.)

Markierungen:

Biotin-11-dUTP (Blugene® nonradioaktive DNA detection kit, Best.-Nr. 8279SA der Fa. Gibco/BRL)

Digoxigenin-dUTP (hergestellt und markiert nach Beispiel 4 und 14).

$^{35}$S-dATP (Amersham-Buchler, Best. Nr. SJ1304), spezifische Aktivität 1200 Curie/mmol

Hybridisierungs- und Waschbedingungen gemäß J. mol. Biol. 3 (1961) 585 ff.

Zellen der genannten Zellinien werden auf HCl gereinigten (0,1 N HCl, 10 min bei 100 °C in phosphate buffered saline (PBS)) gewaschen und anschließend auf UV-bestrahlten Objektträgern in Plastikkulturschalen ausgesät. Als Kulturmedium wurde hierzu minimal essential medium (MEM) mit 5 % an fötalem Kälberserum verwendet (MEM, PBS, SSC vgl. Science 130 (1959) 432 ff).

Nach Ausbildung eines halbkonfluenten Zellrasens wurden die Objektträger den Kulturschalen entnommen und mehrfach mit PBS gewaschen.

Dann schloß sich die Fixierung der Zellen auf der Glasoberfläche an. Hierzu wurde 4 % Paraformaldehyd verwendet (5 min bei Raumtemperatur, dann Waschen in 2 x SSC).

Die Hybridisierung erfolgte unter silikonisierten Deckgläsern. Hierzu wurde die DNA der HPV-Probe in Hybridisierungslösung (nicht-radioaktive Probe: 20 ng/Hybridisierungsbereich, Isotop-markierte Probe: 3 ng mit fünfmal $10^5$ cpM/Hybridisierungsbereich) auf den Zellrasen gegeben. (Die Vorhybridisierung erfolgte wie in Beispiel 19 beschrieben). Nach Auflegen des Deckgläschens wurde dieses versiegelt (z.B. mit Fixogum$^R$ von Marabu). Die Denaturierung der DNA in den Zellen und der DNA-Probe erfolgte dann durch Auflegen des Objektträgers auf einen Heizofen für 3 bis 5 Minuten. Hierbei wurde eine Temperatur von 90 - 92 °C auf dem Objektträger mittels eines Temperaturfühlers gemessen. Die Objektträger werden dann kurz auf Eis gelegt und anschließend über Nacht in einer feuchten Kammer (2 x SSC) inkubiert.

Nach Ablösen der Deckgläser wurde mit 2 x SSC, 0,1 % SDS, 2 x 15 min bei Raumtemperatur und 0,2 x SSC, 0,1 % SDS, 2 x 15 min bei 52 ° C gewaschen.

Im Falle der nicht-radioaktiven Systeme wurde dann nach Beispiel 21 bzw. im Biotinsystem (vgl. Arbeitsanleitung des BRL-Kits: Blocken mit 3 % Rinderserumalbumin, Zugabe eines Konjugats aus Streptavidin und alkalischer Phosphatase sowie Substratmischung) gearbeitet. Im Digoxigenin-System wurde entsprechend Beispiel 21 verfahren. Die Enzymreaktion wurde bis zu 48 Stunden beobachtet.

Als Probe wurde Digoxigenin-markiertes Subfragment des Genoms von HPV TYP 18 (PNAS 80 (1983) 3812-3815, New Engl. J. of Med. 310 (1984) 880-883) verwendet. Es zeigt sich, daß mit dem Digoxigeninsystem in HeLa-Zellen deutlich die Anfärbung des Kernbereichs zu erkennen ist. Nach DNase-Verdau vor Hybridisierung bleibt nur ein geringer unspezifischer Hintergrund. Das Weglassen der markierten Probe führt zu keiner Anfärbung. Auch bei SiHa-Zellen, die lediglich zwei bis drei Kopien HPV pro Zelle ins Genom integriert haben, gelingt der HPV-Nachweis über Nacht. Auch bei diesen Zellen wird vorwiegend der Kernbereich angefärbt. DNase-Verdau führt zu geringem Hintergrund. Das Weglassen der markierten Probe führt zu ungefärbten Zellen.

Anders ist die Situation bei Verwendung von Biotin-markierten Proben. Das Biotin-System führt zwar zu einer schwachen Anfärbung von HeLa-Zellen mit SiHa-Zellen, wird jedoch vorwiegend unspezifisch das Cytosol angefärbt. Der Kernbereich ist nur wenig angefärbt. Die Unspezifität des Biotin-Nachweissystems zeigt sich auch darin, daß die gleiche Anfärbung des Cytosols auch ohne Biotin-markierter Probe, jedoch nach Zugabe allein eines Streptavidin-alkalische-Phosphatase-Konjugats und des entsprechenden Farbstoff-Systems erfolgt.

Mit radioaktiy markierten $^{35}$S-Proben ist zwar der HPV-Nachweis in HeLa- und SiHa-Zellen möglich, jedoch ist dazu eine Autoradiographie von 2 - 3 Wochen notwendig.

Bei in situ-Hybridisierung an fixierten Zellen hat das erfindungsgemäße Verfahren gegenüber dem Verfahren unter Verwendung von radioaktiy markierten Proben den Vorteil der Zeitersparnis gegenüber dem Verfahren unter Verwendung von Biotin-markierten probes den Vorteil der höheren Sensitivität und deutlich höherer Spezifität.

Neben in situ-Hybridisierung in fixierten Zellen ist mit dem erfindungsgemäßen System ebenfalls ein HPV-Nachweis in Gewebsabstrichen, z.B. in HPV-infiziertem Gewebe aus dem Vagina-Bereich, möglich.

Beispiel 25

Nachweis von SUP 6 tRNA durch Colony-Hybridisierung

Ein 750 bp BamHI-Fragment von SUP 6 tRNA (Science 209 (1978), 1396-1460) aus Saccaromyces cerevisiae wurde in pUC19 (Gene 133 (1985), 103-119) kloniert und in E.coli HB 101 (DSM 1607) transformiert. Als Kontrolle wurde der pUC19-Vektor allein in E.coli HB 101 transformiert. Danach wurden abwechselnd Insert-haltige Bakterien neben Kontroll-Transformanten auf Nitrocellulose aufgestrichen. Nach Inkubation für 5 Stunden bei 37 ° C auf Agarplatten mit Nährmedium wurden die E.coli-Zellen alkalisch behandelt, gewaschen und die freigesetzte DNA durch cross-linking bei 354 nm die Membran fixiert. Anschließend erfolgte wiederum Waschen für 3 Stunden bei 50 ° C, Vorhybridisierung und Hybridisierung analog Beispiel 19 mit Digoxigenin-markiertem SUP tRNA-BamHI-Fragmenten (Herstellung analog Beispiel 14). Für die anschließende Detektion ist eine Stunde ausreichend.

Die Insert-haltigen Bakterien geben jeweils eindeutige Signale, die Kontrolltransformanten werden nicht detektiert. Längere Detektionszeiten verstärken die positiven Signale, die Kontrolltransformanten bleiben jedoch auch bei längerer Detektion hintergrundfrei.

Beispiel 26

In situ-Hybridisierung von Lambda Plaques

Lambda gt10-Phagen (Mol.Gen.Genet. 150 (1977), 53) mit einer vollständigen cDNA von humaner Urokinase als Insert wurden auf Indikatorplatten inkubiert, so daß auf einer Platte ca. 500 einzelne Plaques sichtbar waren. Auf eine weitere Platte wurde eine Mischung aus Urokinase-Rekombinaten und einer gleichen Phagenmenge, bestehend aus einer heterogenen Population einer Genbank ausplattiert. Diese Platte diente als Kontrolle, denn nur die Urokinase-Rekombinanten sollten mit einer Urokinase cDNA als Hybridisierungsprobe ein positives Signal geben. Von allen Platten wurden zwei Nitrocellulose-Abklatsche, wie in Science 196 (1977), 180 beschrieben, hergestellt und hybridisiert.

Die Probe wurde dadurch hergestellt, daß 12 ug eines Plasmids, welches die humane Urokinase cDNA enthält, mit PstI verdaut wurde. Dabei entsteht ein 1,1 Kb-Teilfragment der cDNA, welches durch Elektrophorese eluiert wurde (Ausbeute etwas 2 ug).

Die Markierung des Teilfragments erfolgt analog Beispiel 14. Die Vorhybridisierung und Hybridisierung der Filter erfolgt analog Beispiel 19. Die weiteren Detektionsschritte erfolgen analog Beispiel 21.

Es zeigte sich, daß die Filter der Platten, die nur Urokinase-rekombinante Phagen enthielten, ein positives Signal für alle Plaques gaben, die auch im zweiten Abklatsch gut sichtbar waren. Im Falle der Mischung waren lediglich so viele positive Signale zu sehen, wie rekombinante Phagen zugesetzt wurden. Die Lambda-Phagen mit anderen Inserts gaben kein positives Signal.

## Beispiel 27

Northern-blot-Hybridisierung (Nachweis von Actin-mRNA aus Hefe)

Das erfindungsgemäße System ist auch zum sensitiven und spezifischen Nachweis von RNA in Northern-blots geeignet. Um dies zu demonstrieren, wurde Actin-mRNA in Gesamt-Hefe-RNA (Saccharomyces cerevisiae) nachgewiesen. Das Actin-Gen (PNAS 77 (1980), 3912-3916) besteht aus zwei Exons und einem Intron. Bei der Transkription ensteht zunächst Vorläufer-mRNA (1679 Basen). Nach Capping, Splicing und Polyadenylierung (Durchführung wie in Meth. in Enzymol. 65 (1980) 380-391 und PNAS USA 77 1980) 5201-5205 beschrieben) entsteht gereifte mRNA mit einer Länge von ca. 1400 Basen (1370 Basen + PolyA).

Danach erfolgt Transfer auf Nitrocellulose wie in T. Maniatis et al. "Molecular cloning" Cold Spring Harbor Laboratory, Cold Spring Harbor, New York (1982) beschrieben. Die Vor- und Haupthybridisierung und Detektion (18 Stunden) erfolgt wie in Beispiel 19 beschrieben. Zur Detektion wurde eine Digoxigenin-markierte Actin-spezifische Probe, welche aus einem 2,3 kB-Actin-EcoRI/HindIII-Fragment des Actin-Gens bestand, verwendet.

## Beispiel 28

Nachweis von Hepatitis B-Virus (HBV) in Humanseren durch Slot-blot-Analyse

Das Hepatitis B-Virus hat ein ca. 32 kb großes zirkuläres Genom, das aus zwei linearen komplementären Strängen aufgebaut ist. Das 3′-Ende des einen Strangs ist variabel. Die 5′-Enden der beiden komplementären Stränge sind gegeneinander versetzt. Für den HBV-Nachweis auf DNA-Basis wurde ein Digoxigenin-markiertes 1,8 kb BamHI-Fragment (Herstellung nach Beispiel 14) als Probe verwendet, das den doppelsträngigen Bereich Core-Antigen sowie Teile des Prae-S1-, Prae-S2- und X-Antigens enthält (Position 1,4 - 3,2 kb). Parallel zum Digoxigeninnachweis wurden Biotin - (vgl. Beispiel 24) und $^{32}$P-markierte Proben (Markierung analog Arbeitsanweisung des BRL-Kits, vgl. Beispiel 24) verwendet.

Die untersuchten Humanseren sind auf Basis der immunologischen Marker $HB_sAg$, $HB_cAg$, Anti-$HB_s$ und Anti-$HB_c$ als positiv oder negativ eingestuft.

Serum-Vorbereitung:

Für eine Doppelbestimmung werden 100 $\mu$l zentrifugiertes Serum mit 300 $\mu$l 0,5 M NaOH versetzt. Nach 5 minütiger Inkubation bei Raumtemperatur wird erneut 5 min zentrifugiert. Jeweils 200 $\mu$l des Überstands werden in einen Kammschlitz des Minifold$^R$-Geräts pipettiert und dann Vakuum angelegt.

Vorbereitung des Nylon-Filters:

Biodyne Nylon-Membran (1,2 $\mu$m Porengröße) (Pall Bio Support Div. Glen Cove, N.Y.) wird auf Kammergröße zugeschnitten und folgendermaßen vorbereitet:
5 min in destilliertem Wasser eingelegt
15 min in 10 x SSC
5 min unter Wärmelampe auf Whatman$^R$-Papier getrocknet.
Auflegen auf 10 x SSC-getränktes Whatman$^R$-Papier und Einlegen in Minifold$^R$-Apparatur.

Die Probensammelplatte wird dann auf das Filter gelegt, festgeklemmt, und anschließend werden die Seren gespottet.

Weiterbehandlung des Filters:

Die Filter werden aus dem Minifold$^{R}$-Gerät entnommen und für jeweils 5 min auf 10 x SSC, 5 x SSC und 1 x SSC aufgelegt. Anschließend wird unter einer Wärmelampe getrocknet und für 2 Stunden bei 80 °C im Vakuum gebacken.

Die Vorhybridisierung und Hybridisierung erfolgt analog Beispiel 19.

Die Herstellung der radioaktiven Probe erfolgt wie in der Arbeitsanleitung des Sp6/T7 Transcription kit von BOEHRINGER MANNHEIM GMBH, Best.-Nr. 999644, beschrieben.

Praehybridisierung, Hybridisierung, Waschen und Entwikkeln für die $^{32}$p-Probe erfolgt wie in Maniatis (supra) beschrieben.

Es zeigt sich, daß mit dem erfindungsgemäßen Verfahren Signale im Slot-blot nur mit den positiven, nicht jedoch mit den negativen Seren erhalten werden. Die Kontrollreaktionen mit immunisiertem Serum sind ebenfalls negativ. Der Vergleich mit entsprechendem raktioaktiven Nachweis zeigt analoge Spezifität. Im Biotin-System dagegen ist deutlich erhöhter Hintergrund zu sehen.

Der HPV-Nachweis mit dem erfindungsgemäßen Verfahren ist sehr empfindlich. In einer Verdünnungsreihe können HPV-Sequenzen bis zu einer Serumverdünnung von $10^{-4}$ bis $10^{-5}$ nachgewiesen werden. Dies entspricht der Sensitivität im radioaktiven System. DIe entsprechenden Negativ-Seren ergeben im nicht-radioaktiven System bei keiner Verdünnung ein Signal. Auch in der Verdünnungsreihe ist im Biotinsystem ein deutlicher Hintergrund zu sehen.

Beispiel 29

Digoxigenin-markiertes dUTP wird in der polymerase chain reaction (PCR, analog EP-A 0200362) zur Markierung amplifizierter DNA eingesetzt.

Als sample wird verwendet: Plasmid pBR 322

Primer 1: 5′-GCTCCCTCGTGCGCTCTCCTGT-3′

Primer 2: 5′-CCGCCTACATACCTCGCTCTGC-3′

10 pg sample, 300 ng primer 1, 300 ng primer 2, je 1 mmol/l dATP, dCTP, dGTP, dTTp, 0,1 mmol/l Digoxigenin-dUTP in 67 mmol/l Tris-HCl pH 8,8, 6,7 mmol/l $MgCl_2$, 16,6 mmol/l Ammoniumsulfat, 1 mmol/l Mercaptoethanol und 0,17 mg/ml Rinderserumalbumin werden 2 min bei 95 °C denaturiert und 3 min bei 40 °C hybridisiert. Danach werden 6 U Thermus aquaticus DNA-Polymerase zugegeben und 3 min bei 65 °C inkubiert (Polymerisierung). (Das Volumen der Reaktion beträgt 50 Mikroliter, die Konzentrationsangaben beziehen sich auf dieses Reaktionsvolumen). Insgesamt wurden 25 Zyklen (Denaturierung, Hybridisierung, Polymerisierung) durchgeführt.

Anschließend werden je 1/5 der Reaktionsansätze in 0,8 % Agarose-Gel aufgetrennt und nach der Methode von Southern (J.Mol.Biol. 98 (1975) 503) auf Nylonmembranen (z.B. Hybond-N, Amersham) transferiert. Alternatiy werden Verdünnungen von 1:10 bis 1:$10^5$ auf Nylonmembranen aufgetropft und fixiert. Blockierung der Filter, Konjugatreaktionen und Färbung werden analog Beispiel 21 durchgeführt.

## Patentansprüche

1. Verfahren zum Nachweis von Nukleinsäuren definierter Sequenz durch Hybridisierung mit einer komplementären Nukleinsäure-Sonde, die über eine chemische Bindung mindestens ein Hapten als Markierung gebunden enthält, **dadurch gekennzeichnet,** daß man als Hapten ein Steroid verwendet, das an mindestens eine Position der Nukleinsäure-Sonde, die nicht an der Wasserstoffbrückenbildung beteiligt ist, über eine Brücke von mindestens 4 Atomen Länge gebunden ist und die hybridisierte Sonde über einen seinerseits markierten Anti-Hapten-Antikörper nachweist.

2. Verfahren nach einem der Ansprüche 1,
**dadurch gekennzeichnet,**
daß man als Steroid Digoxigenin oder Digoxin verwendet.

3. Verfahren nach Anspruch 1 oder 2,
**dadurch gekennzeichnet**,
daß das Hapten über die Brücke an die $C_5$-Position von Uracil oder Cytosin oder an die $C_8$-Position von Adenin oder Guanin gebunden ist.

**4.** Verfahren nach Anspruch 1 oder 2,
**dadurch gekennzeichnet,**
daß das Hapten über die Brücke an die 2′-Position der Ribose gebunden ist.

**5.** Verfahren nach einem der Ansprüche 1 bis 4,
**dadurch gekennzeichnet,**
daß eine Nukleinsäure-Sonde verwendet wird, deren Hapten in die Nukleinsäure-Sonde enzymatisch mit Hilfe von DNA-Polymerasen, RNA-Polymerasen, Reversen Transkriptasen oder Terminalen Transferasen und entsprechenden Hapten-modifizierten Desoxy- oder Ribonukleosidtriphosphat-Substraten eingebaut wurde.

**6.** Verfahren nach einem der Ansprüche 1 bis 4,
**dadurch gekennzeichnet,**
daß eine Nukleinsäure-Sonde verwendet wird, deren Hapten in die Nukleinsäure-Sonde photochemisch mit Hilfe von Photo-Hapten eingebaut wurde.

**7.** Verfahren nach einem der Ansprüche 1 bis 4,
**dadurch gekennzeichnet,**
daß eine Nukleinsäure-Sonde verwendet wird, deren Hapten in die Nukleinsäure-Sonde chemisch im Rahmen der Oligo-Desoxyribonukleotid-Synthese durch Einbau geschützter, mit substituierbaren Aminofunktionen modifizierter Nukleosidphosphoamidite und - nach Entfernung der Schutzgruppen - durch Umsetzung des modifizierten Oligo-Desoxyribonukleotids mit aktivierten Estern, Amiden oder Ethern des Haptens eingebaut wurde.

**8.** Verfahren nach einem der Ansprüche 1 bis 7,
**dadurch gekennzeichnet,**
daß als Markierung des Anti-Hapten-Antikörpers eine Enzymmarkierung, radioaktive Markierung, Fluoreszenzmarkierung oder (Bio-)Lumineszenzmarkierung verwendet wird.

**9.** Verfahren nach einem der vorhergehenden Ansprüche,
**dadurch gekennzeichnet,**
daß die nachzuweisenden Nukleinsäuren durch in situ-Hybridisierung in auf Objektträgern fixierten Zellen nachgewiesen werden.

**10.** Verfahren nach einem der Ansprüche 1 bis 8,
**dadurch gekennzeichnet,**
daß die nachzuweisenden Nukleinsäuren durch in situ-Hybridisierung in Gewebsabstrichen nachgewiesen werden.

**11.** Verfahren zum Nachweis von Nukleinsäuren
**dadurch gekennzeichnet,**
daß man die nachzuweisende Nukleinsäure nach Denaturierung

(a) mit mindestens zwei Primern, von welchen ein erster in seiner Sequenz komplementär zu einer Teilsequenz eines Stranges der nachzuweisenden Nukleinsäure und ein weiterer identisch mit einer anderen Teilsequenz desselben Stranges der nachzuweisenden Nukleinsäure ist, zur Hybridisierung bringt,
(b) mit Polymerase, Desoxyribonukleotiden und mindestens einem Desoxyribonukleotid, das chemisch gebunden ein Steroid als Hapten enthält, welches an mindestens eine Position des Desoxyribonukleotids, die nicht an der Wasserstoffbrückenbindung beteiligt ist, über eine Brücke von mindestens 4 Atomen Länge gebunden ist, behandelt,
(c) den Zyklus, bestehend aus Denaturierung, Hybridisierung und Polymerisierung, mindestens einmal wiederholt und
(d) die entstandene markierte Nukleinsäure über einen seinerseits markierten Antihapten-Antikörper nachweist.

**12.** Verfahren zum Nachweis von Nukleinsäuren
**dadurch gekennzeichnet,**
daß man die nachzuweisende Nukleinsäure nach Denaturierung

(a) mit mindestens zwei Primern, von welchen ein erster in seiner Sequenz komplementär zu einer Teilsequenz eines Stranges der nachzuweisenden Nukleinsäure und ein weiterer identisch mit einer anderen Teilsequenz desselben Stranges der nachzuweisenden Nukleinsäure ist, zur Hybridisierung bringt,
(b) mit Desoxyribonukleotiden und einer Polymerase behandelt,
(c) anschließend den Zyklus bestehend aus Denaturierung, Hybridisierung und Polymerisierung mindestens einmal wiederholt,
(d) abschließend einen Zyklus in Gegenwart von mindestens einem Desoxyribonukleotid durchführt, welches chemisch gebunden ein Steroid als Hapten enthält, welches an mindesten. eine Position des Desoxyribonukleotids, die nicht an der Wasserstoffbrückenbindung beteiligt ist, über eine Brücke von mindestens 4 Atomen Länge gebunden ist, und
(e) die entstandene markierte Nukleinsäure über einen seinerseits markierten Antihapten-Antikörper nachweist.

**13.** Verfahren nach Anspruch 11 oder 12,
**dadurch gekennzeichnet,**
daß nach Ablauf der Zyklen eine zur nachzuweisenden Nukleinsäure komplementäre, an eine Festphase immobilisierte Nukleinsäure zugegeben, eine Hybridisierungsreaktion durchgeführt und nach Trennung von fester und flüssiger Phase die Markierung in der Festphase bestimmt wird.

**14.** Verfahren nach einem der Ansprüche 11 bis 13,
**dadurch gekennzeichnet,**
daß man als Polymerase die Taq-DNA-Polymerase verwendet.

**15.** Derivatisierte Nukleinsäure, welche über eine chemische Bindung mindestens ein Hapten als Markierung gebunden enthält, dadurch gekennzeichnet, daß das Hapten ein Steroid ist, das an mindestens eine Position der Nukleinsäure, die nicht an der Wasserstoffbrückenbildung beteiligt ist, über eine Brücke von mindestens 4 Atomen Länge gebunden ist.

**16.** Nukleinsäure nach Anspruch 15, dadurch gekennzeichnet, daß das Steroid Digoxigenin ist.

**17.** Verwendung einer Nukleinsäure nach Anspruch 15 als Sonde zum Nachweis von komplementären Nukleinsäuren.

**18.** Desoxyribo- oder Ribo-Nukleosidtriphosphat, welches chemisch gebunden ein Hapten als Markierung gebunden enthält, dadurch gekennzeichnet, daß das Hapten ein Steroid ist, das über eine Brücke von mindestens 4 Atomen Länge an eine Position, die nicht an der Wasserstoffbrückenbildung beteiligt ist, gebunden ist.

**19.** Desoxyribo- oder Ribo-Nukleosidtriphosphat nach Anspruch 18, dadurch gekennzeichnet, daß das Steroid Digoxigenin ist.

**20.** Verfahren zur Herstellung einer derivatisierten Nukleinsäure nach Anspruch 15, wobei das Hapten enzymatisch mit Hilfe von DNA-Polymerasen, RNA-Polymerasen, reversen Transkriptasen oder terminalen Transferasen und einem Desoxyribo- oder Ribo-Nukleosidtriphosphats nach Anspruch 18 eingebaut wird.

**21.** Verfahren zur Herstellung einer derivatisierten Nukleinsäure nach Anspruch 15, wobei im Rahmen der Oligodesoxyribonukleotidsynthese nach der Phosphittriestermethode neben den geschützten Nukleosidphosphoramiditen geschützte mit substituierbaren Aminofunktionen modifizierte Nukleosidphosphoramidite gezielt in den Oligodesoxyribonukleotid-Einzelstrang eingebaut werden, die Schutzgruppen entfernt werden und das entstandene einzelsträngige Oligodesoxyribonukleotid mit einem aktivierten Ester, Amid oder Äther des Steroids umgesetzt wird.

## Claims

**1.** Process for the detection of nucleic acids of definite sequence by hybridisation with a complementary nucleic acid probe which, as labelling, contains at least one hapten bound via a chemical bond, characterised in that, as haptene, one uses a steroid which is bound via a bridge of at least 4 atoms length to at least one position of the nucleic acid probe which is not involved in hydrogen bridge formation and detects the hybridised probe via an anti-hapten antibody which is itself labelled.

**2.** Process according to claim 1, characterised in that one uses digoxigenin or digoxin as steroid.

**3.** Process according to claim 1 or 2, characterised in that the hapten is bound via the bridge to the $C_5$-position of uracil or cytosine or to the $C_8$-position of adenine or guanine.

**4.** Process according to claim 1 or 2, characterised in that the hapten is bound via the bridge to the 2'-position of the ribose.

**5.** Process according to one of claims 1 to 4, characterised in that a nucleic acid probe is used, the hapten of which has been incorporated into the nucleic acid probe enzymatically with the help of DNA polymerases, RNA polymerases, reverse transcriptases or terminal transferases and corresponding hapten-modified desoxy- or ribonucleoside triphosphate substrates.

**6.** Process according to one of claims 1 to 4, characterised in that a nucleic acid probe is used, the hapten of which is incorporated into the nucleic acid probe photochemically with the help of photohapten.

**7.** Process according to one of claims 1 to 4, characterised in that a nucleic acid probe is used, the hapten of which has been incorporated into the nucleic acid probe chemically in the course of the oligodesoxyribonucleotide synthesis by incorporation of protected nucleoside phosphoamidites modified with substitutable amino functions and - after removal of the protective groups - by reaction of the modified oligo-desoxyribonucleotide with activated esters, amides or ethers of the hapten.

**8.** Process according to one of claims 1 to 7, characterised in that, as labelling of the anti-hapten antibody, there is used an enzyme labelling, radioactive labelling, fluorescence labelling or (bio)-luminescence labelling.

**9.** Process according to one of the preceding claims, characterised in that the nucleic acids to be detected are detected by in situ hybridisation in cells fixed on microscope slides.

**10.** Process according to one of claims 1 to 8, characterised in that the nucleic acids to be detected are detected by in situ hybridisation in tissue smears.

**11.** Process for the detection of nucleic acids, characterised in that, after denaturing, the nucleic acid to be detected

(a) is brought to hybridisation with at least two primers of which a first one is, in its sequence, complementary to a part sequence of a strand of the nucleic acid to be detected and a further one is identical with another part sequence of the same strand of the nucleic acid to be detected,

(b) is treated with polymerase, desoxyribonucleotides and at least one desoxyribonucleotide, which, as hapten, contains a steroid chemically bound, which, on at least one position of the desoxyribonucleotide which is not involved in the hydrogen bridge formation, is bound via a bridge of at least 4 atoms length,

(c) the cycle consisting of denaturing, hybridisation and polymerisation is repeated at least once and

(d) the resultant labelled nucleic acid is detected via an antihapten antibody which is itself labelled.

**12.** Process for the detection of nucleic acids, characterised in that, after denaturing, the nucleic acid to be detected

(a) is brought to hybridisation with at least two primers of which a first one is, in its sequence, complementary to a part sequence of a strand of the nucleic acid to be detected and a further one is identical with another part sequence of the same strand,

(b) is treated with desoxyribonucleotides and a polymerase,

(c) subsequently, the cycle consisting of denaturing, hybridisation and polymerisation is repeated at least once,

(d) subsequently, a cycle is carried out in the presence of at least one desoxyribonucleotide, which, as hapten, contains a steroid chemically bound which, on at least one position of the desoxyribonucleotide which is not involved in the hydrogen bridge formation, is bound via a bridge of at least 4 atoms length and

(e) the resulting labelled nucleic acid is detected via an antihapten antibody which is itself labelled.

**13.** Process according to claim 11 or 12, characterised in that, after completion of the cycles, a nucleic acid immobilised on a solid phase, complementary to the nucleic acid to be detected, is added, a hybridisation is carried out and, after separation of solid and liquid phase, the labelling is determined in the solid phase.

**14.** Process according to one of claims 11 to 13, characterised in that one uses the Taq-DNA polymerase as polymerase.

**15.** Derivatised nucleic acid which, via a chemical bond, contains at least one hapten as labelling, characterised in that the hapten is a steroid which, on at least one position of the nucleic acid which is not involved in the hydrogen bridge formation, is bound via a bridge of at least 4 atoms length.

**16.** Nucleic acid according to claim 15, characterised in that the steroid is digoxigenin.

**17.** Use of a nucleic acid according to claim 15 as probe for the detection of complementary nucleic acids.

**18.** Desoxyribo- and ribonucleoside triphosphate which, as labelling, contains a chemically bound hapten, characterised in that the hapten is a steroid which is bound via a bridge of at least 4 atoms length to a position which is not involved in the hydrogen bridge formation.

**19.** Desoxyribo- and ribonucleoside triphosphate according to claim 18, characterised in that the steroid is digoxigenin.

**20.** Process for the production of a derivatised nucleic acid according to claim 15, whereby the hapten is incorporated enzymatically with the help of DNA polymerases, RNA polymerases, reverse transcriptases or terminal transferases and a desoxyribo- or ribonucleoside triphosphate according to claim 18.

**21.** Process for the production of a derivatised nucleic acid according to claim 15, whereby, in the course of the oligodesoxyribonucleotide synthesis according to the phosphite triester method, besides the protected nucleoside phosphoramidites, protected nucleoside phosphoramidites modified with substitutable amino functions are precisely incorporated into the oligodesoxyribonucleotide single strand, the protective groups are removed and the resultant single-stranded oligodesoxyribonucleotide is reacted with an activated ester, amide or ether of the steroid.

## Revendications

**1.** Procédé de détection d'acides nucléiques à séquence définie, par hybridation à l'aide d'une sonde nucléique complémentaire, contenant, comme marqueur, au moins un haptène lié par l'intermédiaire d'une liaison chimique, caractérisé en ce que comme haptène, on utilise un stéroïde, qui est fixé en au moins une position de la sonde nucléique ne participant pas à la formation du pont hydrogène, par l'intermédiaire d'un pont ayant une longueur d'au moins 4 atomes, et que la sonde hybridée permet de détecter un anticorps anti-haptène également marqué.

**2.** Procédé selon la revendication 1, caractérisé en ce que comme stéroïde, on utilise la digoxigénine ou la digoxine.

**3.** Procédé selon la revendication 1 ou 2, caractérisé en ce que l'haptène est fixé par l'intermédiaire du pont dans la position $C_5$ de l'uracile ou de la cytosine, ou dans la position $C_8$ de l'adénine ou de la guanine.

**4.** Procédé selon la revendication 1 ou 2, caractérisé en ce que l'haptène est fixé par l'intermédiaire du pont dans la position 2' de la ribosine.

5. Procédé selon l'une quelconque des revendications 1 à 4, caractérisé en ce qu'on utilise une sonde nucléique dans laquelle l'haptène a été incorporé par voie enzymatique à l'aide d'ADN-polymérase, d'ARN-polymérase, de transcriptase inverse ou de transférase terminale, et de substrats appropriés de triphosphates de désoxy- ou ribonucléoside modifiés par haptène.

6. Procédé selon l'une quelconque des revendications 1 à 4, caractérisé en ce qu'on utilise une sonde nucléique dans laquelle l'haptène a été incorporé par voie photochimique, à l'aide de photohaptènes.

7. Procédé selon l'une quelconque des revendications 1 à 4, caractérisé en ce qu'on utilise une sonde nucléique dans laquelle l'haptène a été incorporé par voie chimique, au cours de la synthèse oligo-désoxyribonucléotidique, par incorporation de nucléosidephosphoamidite modifié par des fonctions amino pouvant être substituées, et - après élimination des groupes protecteurs - par réaction de l'oligo-désoxyribonucléotide modifié avec des esters, amides ou éthers activés de l'haptène.

8. Procédé selon l'une quelconque des revendications 1 à 7, caractérisé en ce que comme marqueur de l'anticorps anti-haptène on utilise un marqueur enzymatique, un marqueur radioactif, un marqueur fluorescent ou un marqueur (bio)luminescent.

9. Procédé selon l'une quelconque des revendications précédentes, caractérisé en ce que les acides nucléiques à détecter ont été détectés par hybridation in situ dans des cellules fixées sur le porte-objet.

10. Procédé selon l'une quelconque des revendications 1 à 8, caractérisé en ce que les acides nucléiques à détecter ont été détectés par hybridation in situ dans des coupes tissulaires.

11. Procédé pour la détection d'acides nucléiques, caractérisé en ce qu'après dénaturation, on soumet l'acide nucléique
(a) à une hybridation avec au moins deux amorces, dont un premier présente une séquence complémentaire d'une séquence partielle d'un brin de l'acide nucléique à détecter, et un autre est identique à une autre séquence partielle du même brin de l'acide nucléique à détecter,
(b) à un traitement avec une polymérase, un désoxyribonucléotide et au moins un désoxyribonucléotide qui contient, comme haptène, un stéroïde lié chimiquement, qui est fixé en au moins une position du désoxyribonucléotide ne participant pas à la liaison par pont hydrogène, par l'intermédiaire d'un pont ayant une longueur d'au moins 4 atomes,
(c) on répète, au moins une fois, le cycle constitué par la dénaturation, l'hybridation et la polymérisation, et
(d) on détecte l'acide nucléique marqué formé par l'intermédiaire d'un anticorps anti-haptène également marqué.

12. Procédé pour la détection d'acides nucléiques, caractérisé en ce qu'après dénaturation, on soumet l'acide nucléique
(a) à une hybridation avec au moins deux amorces, dont un premier présente une séquence complémentaire d'une séquence partielle d'un brin de l'acide nucléique à détecter, et un autre est identique à une autre séquence partielle du même brin de l'acide nucléique à détecter,
(b) à un traitement avec des désoxyribonucléotides et une polymérase,
(c) ensuite on répète, au moins une fois, le cycle constitué par la dénaturation, l'hybridation et la polymérisation, et
(d) enfin, on effectue un cycle en présence d'au moins un désoxyribonucléotide qui contient, comme haptène, un stéroïde lié chimiquement, qui est fixé en au moins une position du désoxyribonucléotide ne participant pas à la liaison par pont hydrogène, par l'intermédiaire d'un pont ayant une longueur d'au moins 4 atomes, et
(e) on détecte l'acide nucléique marqué formé par l'intermédiaire d'un anticorps anti-haptène également marqué.

13. Procédé selon la revendication 11 ou 12, caractérisé en ce qu'après le déroulement des cycles, on ajoute un acide nucléique immobilisé sur une phase solide, complémentaire de l'acide nucléique à détecter, on effectue une réaction d'hybridation et après séparation des phases solide et liquide, on détermine le marqueur dans la phase solide.

**14.** Procédé selon l'une des revendications 11 à 13, caractérisé en ce que comme polymérase, on utilise la Taq-ADN-polymérase.

**15.** Dérivé d'acide nucléique, contenant, comme marqueur, au moins un haptène fixé par l'intermédiaire d'une liaison chimique, caractérisé en ce que l'haptène est un stéroïde qui est fixé en au moins une position du désoxyribonucléotide ne participant pas à la liaison par pont hydrogène, par l'intermédiaire d'un pont ayant une longueur d'au moins 4 atomes.

**16.** Acide nucléique selon la revendication 15, caractérisé en ce que le stéroïde est la digoxigénine.

**17.** Utilisation d'un acide nucléique selon la revendication 15, comme sonde pour la détection d'acides nucléiques complémentaires.

**18.** Triphosphate de désoxy- ou ribo-nucléoside, contenant, comme marqueur, au moins un haptène lié par l'intermédiaire d'une liaison chimique, caractérisé en ce que comme haptène, on utilise un stéroïde, qui est fixé en au moins une position de la sonde nucléique ne participant pas à la formation du pont hydrogène, par l'intermédiaire d'un pont ayant une longueur d'au moins 4 atomes.

**19.** Triphosphate de désoxy- ou ribo-nucléoside selon la revendication 18, caractérisé en ce que le stéroïde est la digoxigénine.

**20.** Procédé de préparation d'un dérivé d'acide nucléique selon la revendication 15, dans lequel l'haptène a été incorporé par voie enzymatique à l'aide d'ADN-polymérase, d'ARN-polymérase, de trancriptase inverse ou de transférase terminale, et d'un triphosphate de désoxy- ou ribo-nucléoside selon la revendication 18.

**21.** Procédé de préparation d'un dérivé d'acide nucléique selon la revendication 15, dans lequel, au cours de la synthèse oligodésoxyribonucléotidique selon le procédé au triester de phosphite, on incorpore dans le brin simple de l'oligodésoxyribonucléotide, en plus des nucléosidephosphoramidites protégés, des nucléosidephosphoramidites protégés, modifiés avec des fonctions amino qui peuvent être substituées, on élimine les groupes protecteurs et l'on fait réagir l'oligodésoxyribonucléotide à brin simple obtenu avec un ester, amide ou éther activé du stéroïde.

FIG.1

Dig-11(16)-dUTP

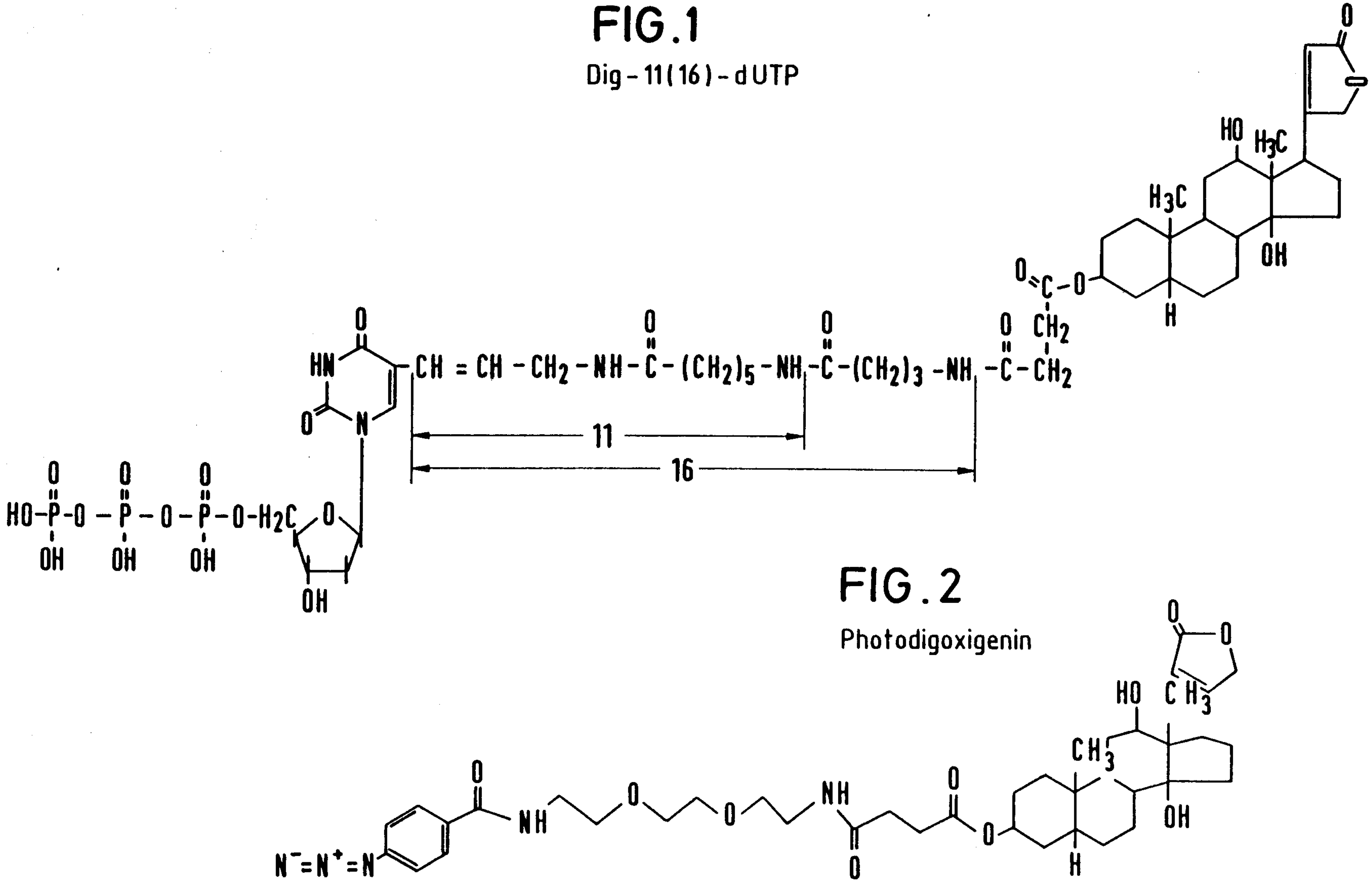

FIG. 2

Photodigoxigenin

# FIG.3

5-Brom-4-chlor-3-indolylphosphat-$Na_2$

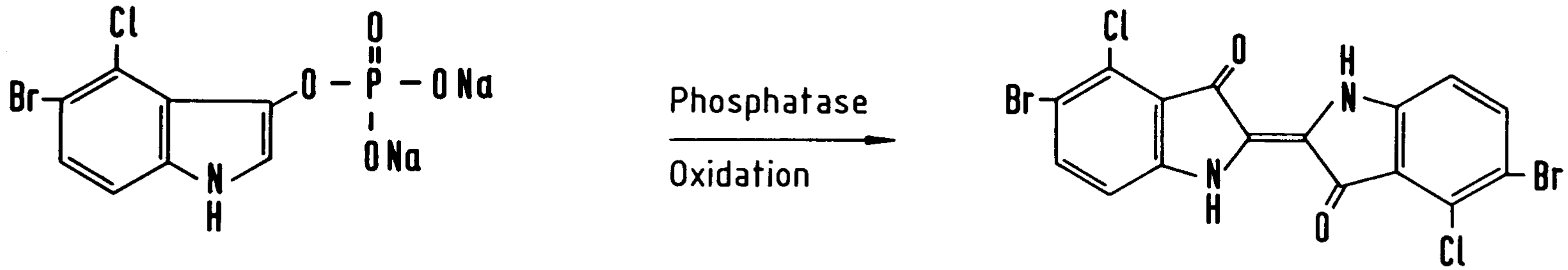


farblos

blau

# zu FIG. 3

Nitrotetrazolium Blue (NBT)
Syn.: 3,3'-(3,3'-Dimethoxy-4,4'-biphenylene)-bis-[5-phenyl-2-(4-nitrophenyl)-tetrazolium chloride]

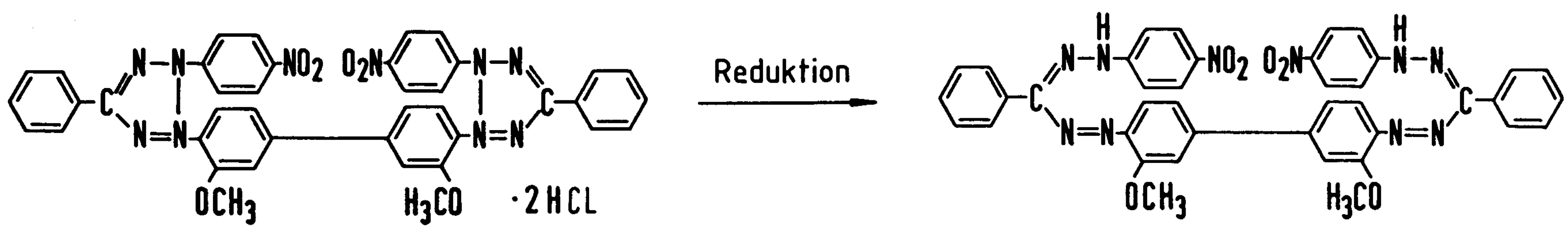


farblos

Tetrazoliumsalz

blau

Formazan

FIG. 4

Hochsensitiver DNA-Nachweis

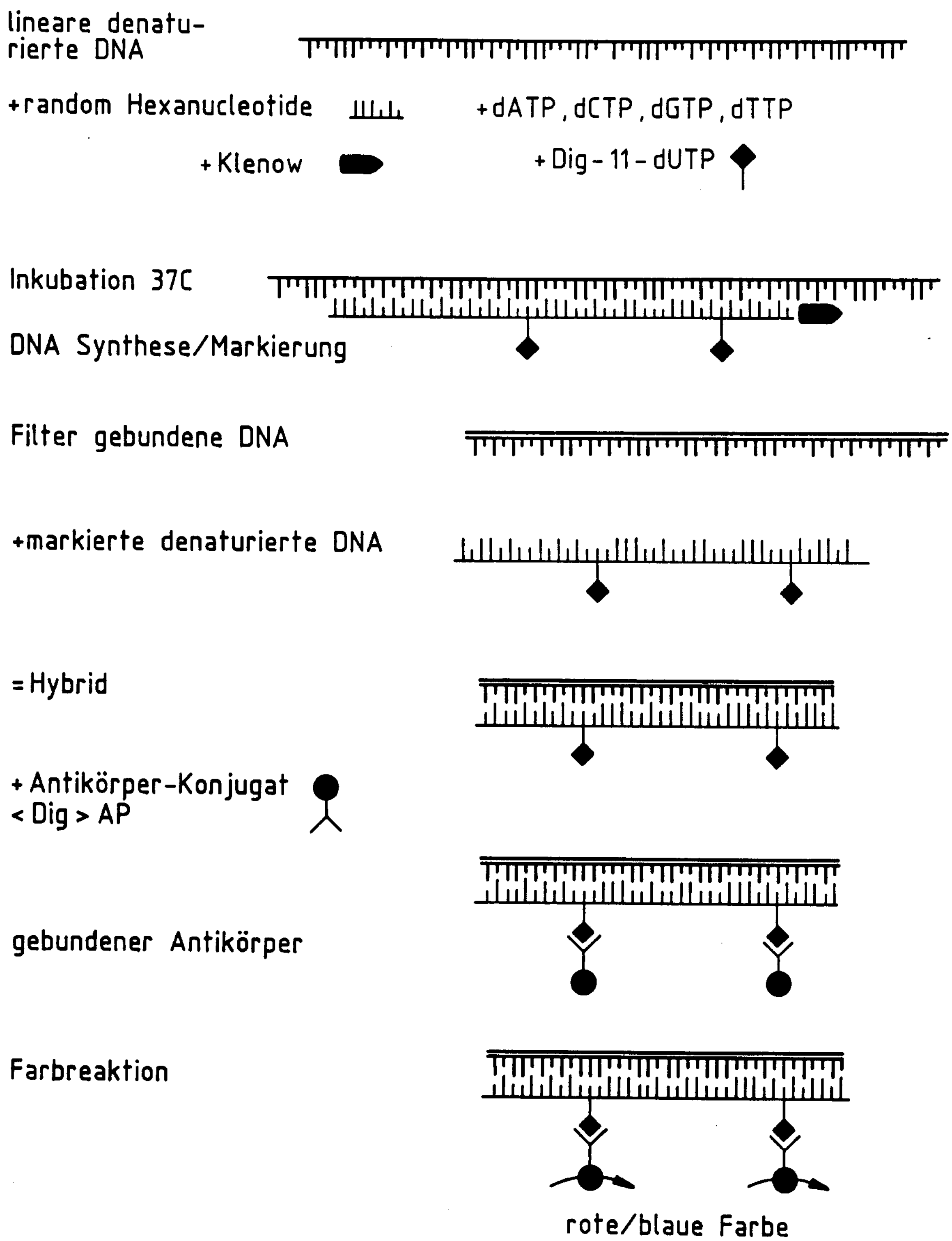

FIG.5

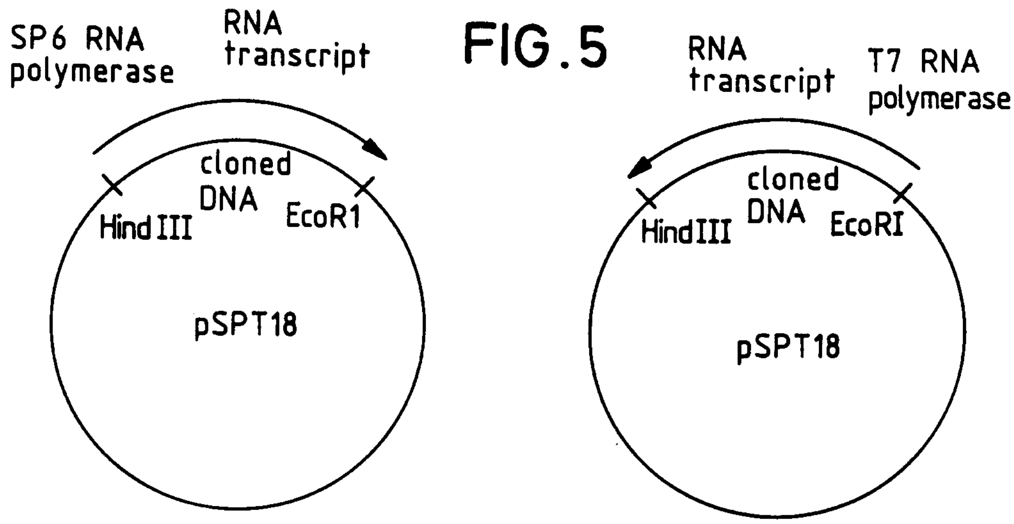
SP6 RNA polymerase
RNA transcript
cloned DNA
Hind III
EcoR1
pSPT18
RNA transcript
T7 RNA polymerase
cloned DNA
HindIII
EcoRI
pSPT18

FIG.6

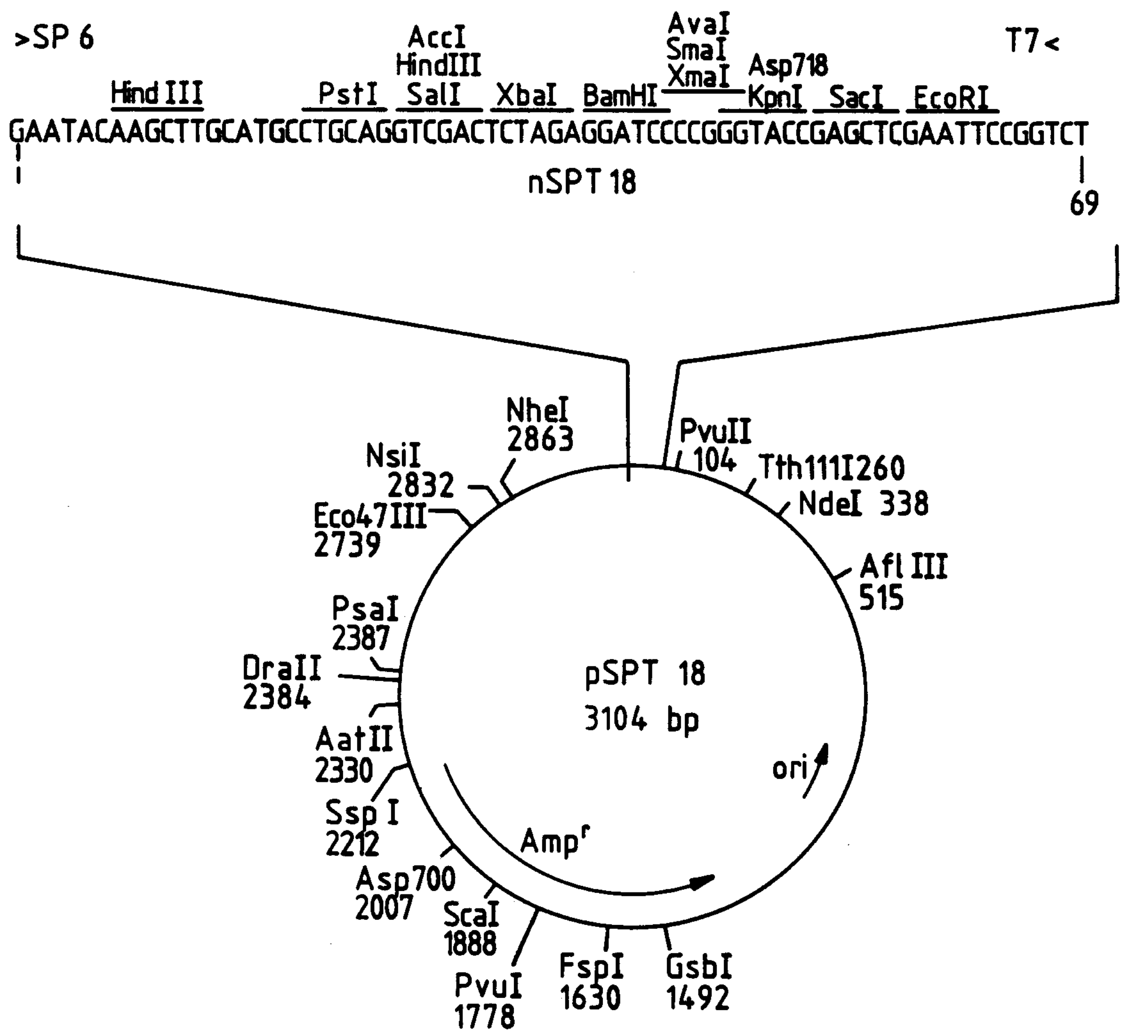
>SP 6
T7<
Hind III
PstI
AccI
HindIII
SalI
XbaI
BamHI
AvaI
SmaI
XmaI
Asp718
KpnI
SacI
EcoRI
GAATACAAGCTTGCATGCCTGCAGGTCGACTCTAGAGGATCCCCGGGTACCGAGCTCGAATTCCGGTCT
nSPT 18
69
NheI 2863
NsiI 2832
Eco47III 2739
PsaI 2387
DraII 2384
AatII 2330
Ssp I 2212
Asp700 2007
ScaI 1888
PvuI 1778
FspI 1630
GsbI 1492
PvuII 104
Tth111I260
NdeI 338
Afl III 515
pSPT 18
3104 bp
ori
Amp^r

# FIG.7

Syntheseschema für Digoxigenin-3-hemisuccinat-/
N'-(4-azidobenzoyl)/-8-amino-3,6-dioxaoctylamid(Photodigoxigenin)

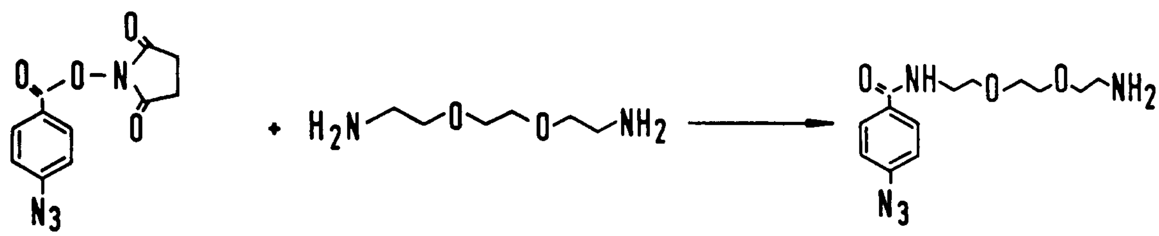

I II III

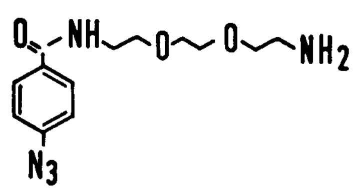

III

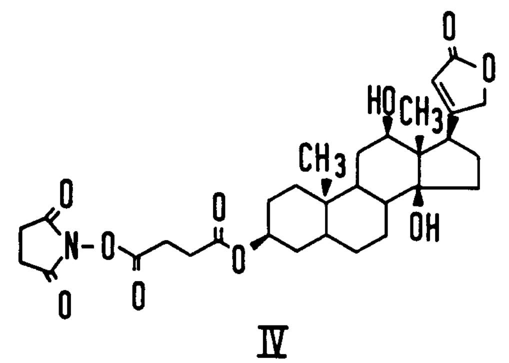

IV

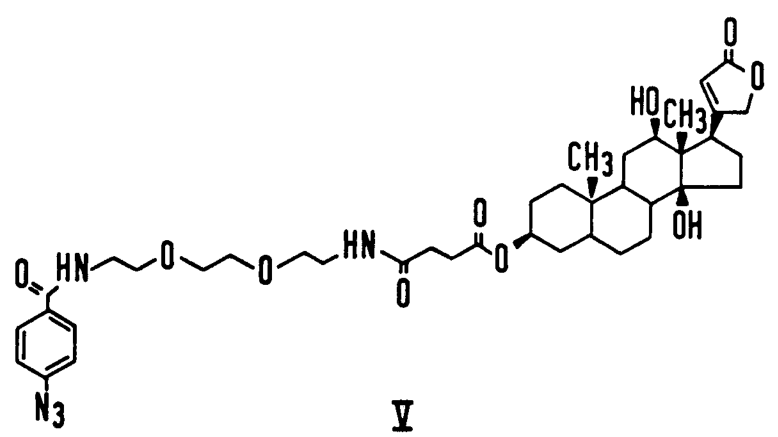

V

# FIG.8

Vergleich der Sensitivitäten von Digoxigenin und Biotin

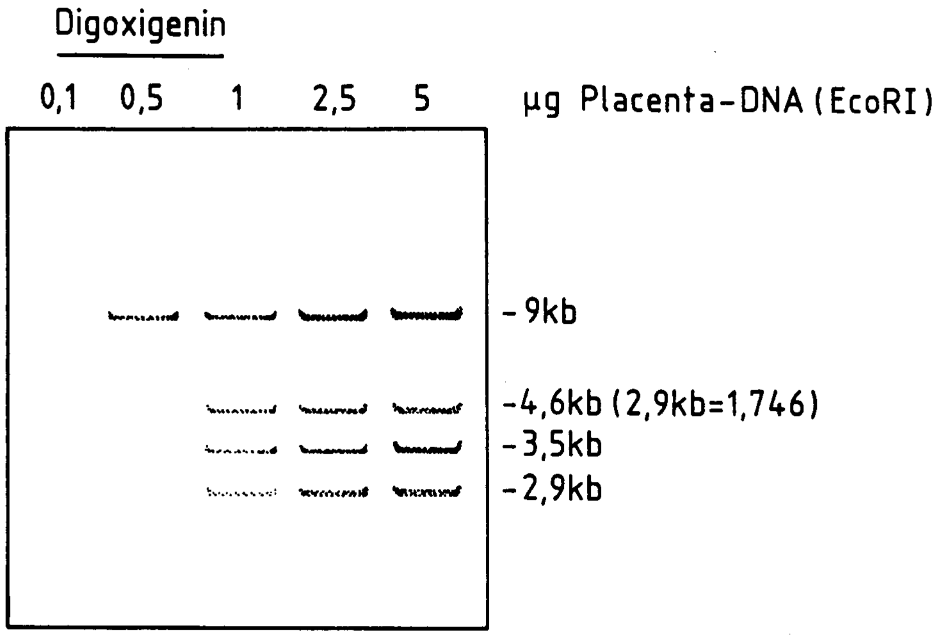


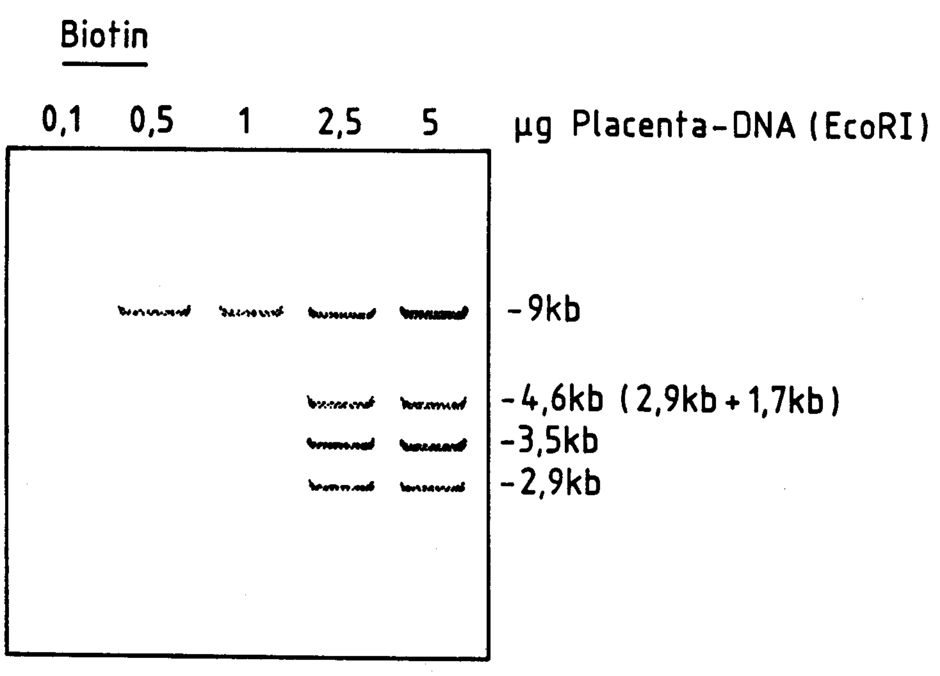

# FIG. 9

Vergleich der Sensitivitäten einer erfindungsgemäßen enzymatischen Markierung (A) mit einer bekannten chemischen Markierung (B)

## FIG. 9A

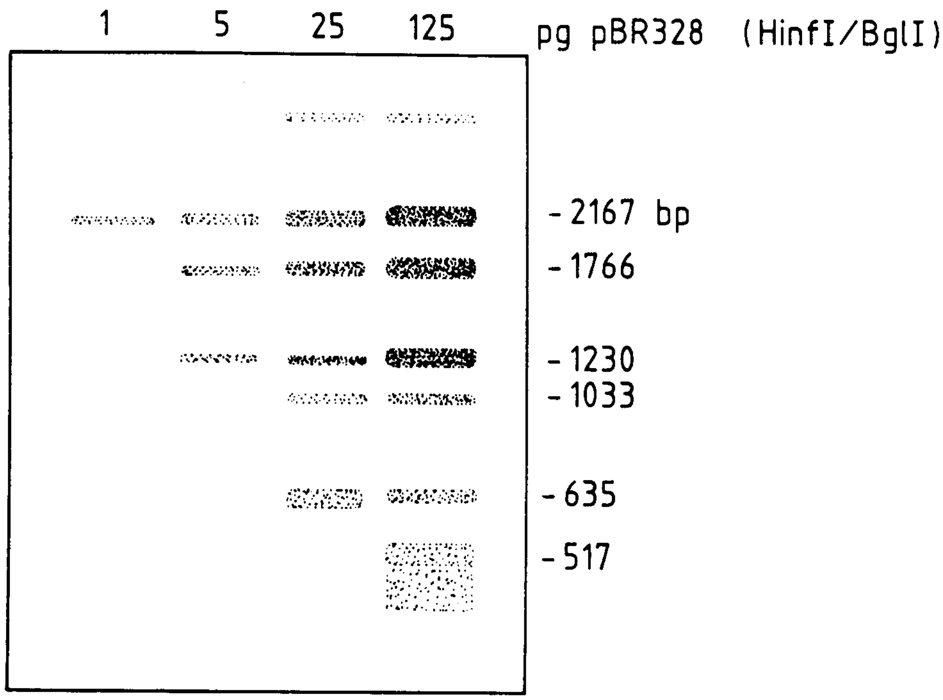


## FIG. 9B

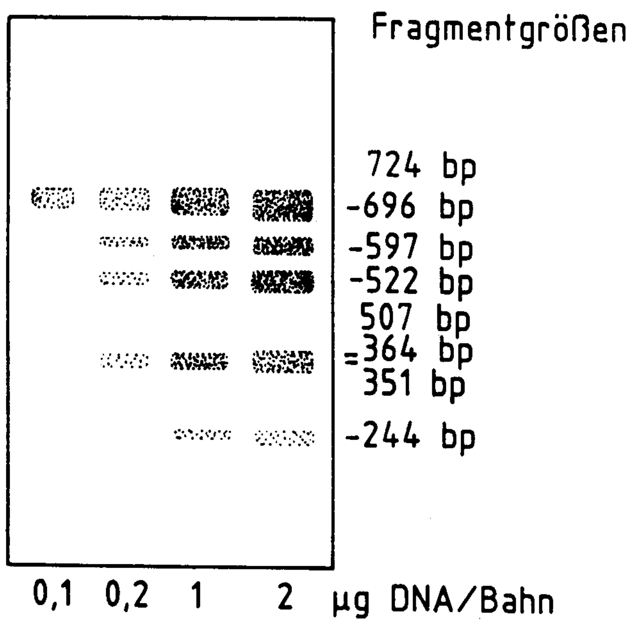

# FIG.10

DNA-Sequenz(A) und Restriktionskarte(B) des Plasmids pSPT18

## FIG.10A

1 GAATACAAGC TTGCATGCCT GCAGGTCGAC TCTAGAGGAT CCCCGGGTAC

51 CGAGCTCGAA TTCCGGTCTC CCTATAGTGA GTCGTATTAA TTTCGATAAG

101 CCAGCTGGGC CTCGCGCGTT TCGGTGATGA CGGTGAAAAC CTCTGACACA

151 TGCAGCTCCC GGAGACGGTC ACAGCTTGTC TGTAAGCGGA TGCCGGGAGC

201 AGACAAGCCC GTCAGGGCGC GTCAGCGGGT GTTGGCGGGT GTCGGGGCGC

251 AGCCATGACC CAGTCACGTA GCGATAGCGG AGTGTATATA CTGGCTTAAC

301 TATGCGGCAT CAGAGCAGAT TGTACTGAGA GTGCACCATA TGCGGTGTGA

351 AATACCGCAC AGATGCGTAA GGAGAAAATA CCGCATCAGG CGCTCTTCCG

401 CTTCCTCGCT CACTGACTCG CTGCGCTCGG TCGTTCGGCT GCGGCGAGCG

451 GTATCAGCTC ACTCAAAGGC GGTAATACGG TTATCCACAG AATCAGGGGA

501 TAACGCAGGA AAGAACATGT GAGCAAAAGG CCAGCAAAAG GCCAGGAACC

551 GTAAAAAGGC CGCGTTGCTG GCGTTTTTCC ATAGGCTCCG CCCCCCTGAC

601 GAGCATCACA AAAATCGACG CTCAAGTCAG AGGTGGCGAA ACCCGACAGG

651 ACTATAAAGA TACCAGGCGT TTCCCCCTGG AAGCTCCCTC GTGCGCTCTC

701 CTGTTCCGAC CCTGCCGCTT ACCGGATACC TGTCCGCCTT TCTCCCTTCG

751 GGAAGCGTGG CGCTTTCTCA ATGCTCACGC TGTAGGTATC TCAGTTCGGT

801 GTAGGTCGTT CGCTCCAAGC TGGGCTGTGT GCACGAACCC CCCGTTCAGC

851 CCGACCGCTG CGCCTTATCC GGTAACTATC GTCTTGAGTC CAACCCGGTA

901 AGACACGACT TATCGCCACT GGCAGCAGCC ACTGGTAACA GGATTAGCAG

951 AGCGAGGTAT GTAGGCGGTG CTACAGAGTT CTTGAAGTGG TGGCCTAACT

1001 ACGGCTACAC TAGAAGGACA GTATTTGGTA TCTGCGCTCT GCTGAAGCCA

1051 GTTACCTTCG GAAAAAGAGT TGGTAGCTCT TGATCCGGCA AACAAACCAC

1101 CGCTGGTAGC GGTGGTTTTT TTGTTTGCAA GCAGCAGATT ACGCGCAGAA

1151 AAAAAGGATC TCAAGAAGAT CCTTTGATCT TTTCTACGGG GTCTGACGCT

1201 CAGTGGAACG AAAACTCACG TTAAGGGATT TTGGTCATGA GATTATCAAA

Fortsetzung **FIG.10A**

1251 AAGGATCTTC ACCTAGATCC TTTTAAATTA AAAATGAAGT TTTAAATCAA

1301 TCTAAAGTAT ATATGAGTAA ACTTGGTCTG ACAGTTACCA ATGCTTAATC

1351 AGTGAGGCAC CTATCTCAGC GATCTGTCTA TTTCGTTCAT CCATAGTTGC

1401 CTGACTCCCC GTCGTGTAGA TAACTACGAT ACGGGAGGGC TTACCATCTG

1451 GCCCCAGTGC TGCAATGATA CCGCGAGACC CACGCTCACC GGCTCCAGAT

1501 TTATCAGCAA TAAACCAGCC AGCCGGAAGG GCCGAGCGCA GAAGTGGTCC

1551 TGCAACTTTA TCCGCCTCCA TCCAGTCTAT TAATTGTTGC CGGGAAGCTA

1601 GAGTAAGTAG TTCGCCAGTT AATAGTTTGC GCAACGTTGT TGCCATTGCT

1651 ACAGGCATCG TGGTGTCACG CTCGTCGTTT GGTATGGCTT CATTCAGCTC

1701 CGGTTCCCAA CGATCAAGGC GAGTTACATG ATCCCCCATG TTGTGCAAAA

1751 AAGCGGTTAG CTCCTTCGGT CCTCCGATCG TTGTCAGAAG TAAGTTGGCC

1801 GCAGTGTTAT CACTCATGGT TATGGCAGCA CTGCATAATT CTCTTACTGT

1851 CATGCCATCC GTAAGATGCT TTTCTGTGAC TGGTGAGTAC TCAACCAAGT

1901 CATTCTGAGA ATAGTGTATG CGGCGACCGA GTTGCTCTTG CCCGGCGTCA

1951 ATACGGGATA ATACCGCGCC ACATAGCAGA ACTTTAAAAG TGCTCATCAT

2001 TGGAAAACGT TCTTCGGGGC GAAAACTCTC AAGGATCTTA CCGCTGTTGA

2051 GATCCAGTTC GATGTAACCC ACTCGTGCAC CCAACTGATC TTCAGCATCT

2101 TTTACTTTCA CCAGCGTTTC TGGGTGAGCA AAAACAGGAA GGCAAAATGC

2151 CGCAAAAAAG GGAATAAGGG CGACACGGAA ATGTTGAATA CTCATACTCT

2201 TCCTTTTTCA ATATTATTGA AGCATTTATC AGGGTTATTG TCTCATGAGC

2251 GGATACATAT TTGAATGTAT TTAGAAAAAT AAACAAATAG GGGTTCCGCG

2301 CACATTTCCC CGAAAAGTGC CACCTGACGT CTAAGAAACC ATTATTATCA

2351 TGACATTAAC CTATAAAAAT AGGCGTATCA CGAGGCCCTT TCGTCTCGCG

2401 CGTTTCGGTG ATGACGGTGA AAACCTCTGA CACATGCAGC TCCCGGAGAC

2451 GGTCACAGCT TGTCTGTAAG CGGATGCCGG GAGCAGACAA GCCCGTCAGG

2501 GCGCGTCAGC GGGTGTTGGC GGGTGTCGGG GCTGGCTTAA CTATGCGGCA

2551 TCAGAGCAGA TTGTACTGAG AGTGCACCAT ATCGACGCTC TCCCTTATGC

2601 GACTCCTGCA TTAGGAAGCA GCCCAGTAGT AGGTTGAGGC CGTTGAGCAC

2651 CGCCGCCGCA AGGAATGGTG CATGCAAGGA GATGGCGCCC AACAGTCCCC

2701 CGGCCACGGG CCTGCCACCA TACCCACGCC GAAACAAGCG CTCATGAGCC

2751 CGAAGTGGCG AGCCCGATCT TCCCCATCGG TGATGTCGGC GATATAGGCG

2801 CCAGCAACCG CACCTGTGGC GCCGGTGATG CCGGCCACGA TGCGTCCGGC

Fortsetzung **FIG .10A**

2851 GTAGAGGATC TGGCTAGCGA TGACCCTGCT GATTGGTTCG CTGACCATTT

2901 CCGGGTGCGG GACGGCGTTA CCAGAAACTC AGAAGGTTCG TCCAACCAAA

2951 CCGACTCTGA CGGCAGTTTA CGAGAGAGAT GATAGGGTCT GCTTCAGTAA

3001 GCCAGATGCT ACACAATTAG GCTTGTACAT ATTGTCGTTA GAACGCGGCT

3051 ACAATTAATA CATAACCTTA TGTATCATAC ACATACGATT TAGGTGACAC

3101 TATA

# FIG. 10B

pSPT18 Restriktionskarte

| Enzyme | Zahl der Schnittstellen | Position der Schnittstellen | | | | |
|---|---|---|---|---|---|---|
| *Ava* II | 2 | 1546 | 1768 | | | |
| *Bgl* I | 2 | 106 | 1528 | | | |
| *Eco* 31I | 2 | 71 | 1469 | | | |
| *Fin* I | 2 | 2699 | 2908 | | | |
| *Hgi* EII | 2 | 335 | 1096 | | | |
| *Sph* I | 2 | 17 | 2674 | | | |
| *Apy* I | 3 | 543 | 664 | 677 | | |
| *Ban* II | 3 | 56 | 2750 | 2764 | | |
| *Bbe* I | 3 | 2688 | 2801 | 2822 | | |
| *Cfr* 10I | 3 | 1488 | 2821 | 2830 | | |
| *Dra* I | 3 | 1274 | 1293 | 1985 | | |
| *Eae* I | 3 | 1796 | 2702 | 2832 | | |
| *Eco* 57I | 3 | 1063 | 2075 | 2977 | | |
| *Eco* RII | 3 | 541 | 662 | 675 | | |
| *Gdi* II | 3 | 1796 | 2701 | 2832 | | |
| *Hae* I | 3 | 528 | 539 | 991 | | |
| *Mme* I | 3 | 730 | 914 | 2966 | | |
| *Nar* I | 3 | 2685 | 2798 | 2819 | | |
| *Tth* 111II | 3 | 1105 | 1112 | 1144 | | |
| *Apa* LI | 4 | 331 | 829 | 2075 | 2572 | |
| *Bsp* HI | 4 | 1235 | 2243 | 2348 | 2742 | |
| *Aha* II | 5 | 1945 | 2327 | 2685 | 2798 | 2819 |
| *Ban* I | 5 | 46 | 1356 | 2684 | 2797 | 2818 |
| *Fok* I | 5 | 201 | 1374 | 1555 | 1842 | 2485 |
| *Mae* I | 5 | 32 | 1010 | 1263 | 1598 | 2864 |
| *Mae* II | 5 | 266 | 1218 | 1634 | 2007 | 2327 |
| *Nsp* I | 5 | 17 | 152 | 519 | 2436 | 2674 |
| *Rsa* I | 5 | 48 | 323 | 1888 | 2564 | 3026 |
| *Sec* I | 5 | 41 | 42 | 675 | 2698 | 2704 |

Enzyme, die nicht spalten

| | | | | |
|---|---|---|---|---|
| *Afl* II | *Apa* I | *Asu* II | *Avr* II | *Bal* I |
| *Bbv* II | *Bcl* I | *Bgl* II | *Bsm* I | *Bsp* MII |
| *Bss* HII | *Bst* EII | *Bst* XI | *Cla* I | *Dra* III |
| *Eco* RV | *Eco* R124 | *Esp* I | *Hpa* I | *Mlu* I |
| *Nco* I | *Not* I | *Nru* I | *Nsi* I | *Pfl* MI |
| *Pma* CI | *Ppu* MI | *Rsr* II | *Sac* II | *Sau* I |
| *Sfi* I | *Sna* I | *Sna* BI | *Spe* I | *Spl* I |
| *Stu* I | *Sty* I | *Xho* I | *Xma* III | |